(19) Europäisches Patentamt
European Patent Office
Office européen des brevets

(11) **EP 2 985 045 B1**

(12) **EUROPÄISCHE PATENTSCHRIFT**

(45) Veröffentlichungstag und Bekanntmachung des Hinweises auf die Patenterteilung:
**11.10.2017 Patentblatt 2017/41**

(51) Int Cl.:
***A61M 1/16*** *(2006.01)*

(21) Anmeldenummer: **15179976.4**

(22) Anmeldetag: **06.08.2015**

(54) **VERFAHREN ZUM EINSTELLEN EINES BLUTFLUSSES IN EINER DIALYSEVORRICHTUNG UND DIALYSEVORRICHTUNG**

METHOD FOR ADJUSTING A BLOOD FLOW IN A DIALYSIS DEVICE AND DIALYSIS DEVICE

PROCEDE DE REGLAGE D'UN FLUX SANGUIN DANS UN DISPOSITIF DE DIALYSE ET DISPOSITIF DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.08.2014 DE 102014111665**

(43) Veröffentlichungstag der Anmeldung:
**17.02.2016 Patentblatt 2016/07**

(73) Patentinhaber: **B. Braun Avitum AG**
**34212 Melsungen (DE)**

(72) Erfinder:
• **KRAUSE, Silvie**
**34212 Melsungen (DE)**
• **Dr. STROHHÖFER, Christof**
**34123 Kassel (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
WO-A1-2013/167264    DE-A1- 10 114 283
DE-A1-102010 047 215    US-A- 5 863 421

EP 2 985 045 B1

**Beschreibung**

Die vorliegende Erfindung betrifft eine extrakorporale Blutbehandlungsmaschine, vorzugsweise Dialysemaschine, die ein Einstellen eines Blutflusses erlaubt. Die extrakorporale Blutbehandlungs-/Reinigungsmaschine, vorzugsweise Dialysemaschine, weist eine Steuereinrichtung oder Steuereinheit zum Einstellen des Blutflusses auf.

Für die extrakorporale Blutbehandlung/Blutreinigung, wie Dialyse, ist die Einstellung des Blutflusses (Qb) von großer Bedeutung für die Behandlungseffizienz. Beispielsweise besitzen Dialysepatienten eine künstliche Punktierungsstelle oder einen künstlichen Zugang zum intrakorporalen Blutgefäßsystem, die entweder aus einem Shunt (einer Verbindung von Vene und Arterie) oder einem Zentral-Venösen-Katheter bestehen kann. An dieser Punktierungsstelle/Gefäßzugang wird das Blut für die Dialysebehandlung aus dem Patientenkörper entnommen. Der Blutfluss wird in der Regel so hoch wie möglich angesetzt, da ein höherer Blutfluss im Allgemeinen mit einer höheren Reinigungsleistung assoziiert wird und damit mit einer besseren Entgiftungsleistung für die Therapie einher geht und/oder zu einer Verringerung der Behandlungsdauer führt.

Während einer Dialysebehandlung können Komplikationen oder gewisse ungewollte Phänomene eintreten. Einige führen dazu, dass die Annahme einer höheren Reinigungsleistung bei einem höheren Blutfluss nicht mehr gilt. Ein Beispiel hierfür ist das Eintreten einer sogenannten Rezirkulation bzw. lokalen Shunt-Rezirkulation. Die Rezirkulation oder Shunt-Rezirkulation (R) ist definiert als das Verhältnis der Flüsse von rezirkuliertem Blut (Qr) und Blutpumpenrate bzw. Gesamtblutfluss (Qb). Als rezirkuliertes Blut wird demnach jenes Blut bezeichnet, welches bereits gereinigt ist und aus der venösen Nadel (Blutrückführleitung) stammt und das zusammen mit noch ungereinigtem Patientenblut direkt zurück in die arterielle Nadel (Blutzuführleitung) befördert (rezirkuliert) wird. Die Rezirkulation bzw. Shunt-Rezirkulation (R) kann als Verhältnis

$$R = Qr / Qb$$

oder in Prozent angegeben werden.

Der medizinisch indizierte Blutfluss ist u. a. abhängig vom Zustand des Patientenzugangs bzw. Shunts. Damit steht der behandelnde Arzt vor der Aufgabe, den Blutfluss bei der extrakorporalen Blutbehandlung einerseits so hoch wie möglich einzustellen, um möglichst die maximale Reinigungsleistung für eine Therapie zu erzielen, andererseits den Blutfluss aber nicht zu hoch anzusetzen, um keine Rezirkulation zu riskieren bzw. die Rezirkulation möglichst klein zu halten.

Aus dem Stand der Technik sind Verfahren und Maschinen für die extrakoporale Blutbehandlung / -reinigung, wie Dialyse, bekannt.

US 5,863,421 A offenbart eine extrakorporale Blutbehandlungsmaschine, bei der der Blutfluss und Blutdruckwerte erfasst und gesteuert werden können.

In der WO 2007/140993 ist eine Vorrichtung zur Steuerung einer extrakorporalen Blutbehandlungsmaschine beschrieben. Bei dieser Blutbehandlungsmaschine wird bei mindestens einer vorgegebenen Flussrate aus einer Gruppe von Flussraten umfassend Blutflussrate Qb, Dialysierflüssigkeitsrate Qd, Ultrafiltratrate Qf und Substituatrate Qs mindestens eine der jeweils anderen Flussraten aus der Gruppe von Flussraten umfassend Blutflussrate Qb, Dialysierflüssigkeitsrate Qd, Ultrafiltratrate Qf und Substituatrate Qs allein auf der Grundlage einer vorgegebenen Abhängigkeit der Clearance K oder Dialysance D von den Flussraten berechnet, bei der die vorgegebene Clearance K oder Dialysance D beibehalten wird.

In der US 3 882 861 ist eine Dialysemaschine beschrieben, mit deren Hilfe der Blutfluss druckgesteuert angepasst wird. Der Blutfluss wird durch eine elektrische Pulsfolge gesteuert, bei der die jeweilige Pulsdauer Änderungen des Unterdrucks bei Änderungen des Blutflusses entspricht. Ein Nachteil dieser Lösung besteht jedoch darin, dass die vorgeschlagene Maschine technisch aufwändig und damit teuer ist. Außerdem wurde festgestellt, dass mit einer solchen Maschine, die ausschließlich druckgesteuert arbeitet, nicht unbedingt gewährleistet werden kann, dass die Reinigungsleistung der Behandlung optimal ist.

In der EP 0 711 182 B1 ist ein System zum Erreichen eines möglichst hohen Clearance-Wertes bezogen auf den Gesamtpatientenkörper beschrieben. Das System hat eine Einrichtung zum Einstellen eines Parameters der Dialyse-Effizienz, eine Einrichtung zum Erfassen einer metabolischen Konzentration, eine Einrichtung zum Ermitteln eines metabolischen Profils in Abhängigkeit von der Parameteränderung und eine Einrichtung zum Vergleichen der gemessenen metabolischen Konzentrationswerte, um einen optimalen Parameter zu bestimmen, mit dem sich eine maximale metabolische Konzentration erzielen lässt.

Ein Nachteil bei diesem Stand der Technik besteht indessen darin, dass die Bestimmung der Harnstoffkonzentration im Abfluss genutzt wird, um eine Aussage zu gewinnen, bei welchem Blutfluss die dialysierflüssigkeitsseitige Toxinkonzentration (hier Harnstoffkonzentration) maximal ist. Diese Messung setzt das Einstellen verschiedener Blutflüsse voraus. Die Messung kann nach Blutflussänderung erst nach Erreichen eines stabilen Wertes, d.h. nach Abschluss des Ausgleichvorganges innerhalb des extrakorporalen Blutleitungssystems der Maschine, dialysierflüssigkeitsseitig erfolgen. Da der Patient während der Messungen jedoch immer weiter dialysiert wird, steht zu bezweifeln, dass der richtige Blutfluss überhaupt ermittelt werden kann, da die Messzeiten sehr hoch/lang sein müssten. Die Vorrichtung und das Verfahren sind daher nicht unbedingt geeignet, um schnell den Blutfluss zu ermitteln, bei dem eine (im Wesentlichen) maximale Toxinentfernung möglich ist.

In der EP 1 083 948 B1 ist ein Verfahren zur Bestimmung der Konzentration von Abfallprodukten, d.h. filtergängigen urämischen Toxinen, in der Dialysierflüssigkeit während einer Dialysebehandlung auf Basis der Transmissionsspektroskopie beschrieben. Die Messung wird spektralphotometrisch durchgeführt, und das Messergebnis wird multipliziert mit dem Durchfluss vom Dialysator, um den Inhalt der Substanz(en) in der ausgangsseitigen Dialyseflüssigkeit zu bestimmen.

Damit lässt sich die Absorbanz eines Stoffgemisches, welches auf Dialysierflüssigkeitsseite existiert, messen. Aus den Daten wird jedoch kein Rückschluss auf den Blutfluss gezogen.

Schließlich sind aus der WO 2013/167264 ein Verfahren sowie ein Vorrichtung zur extrakorporalen Blutbehandlung bekannt, wodurch eine Optimierung einer vorzugebenden Blutflussrate im Sinne einer Maximierung der Austauschleistung eines Dialysators erreicht werden soll. Die Vorrichtung sowie das Verfahren gemäß diesem Stand der Technik sehen hierfür die Ermittlung mindestens einer, vorzugsweise aber einer Mehrzahl von für die extrakorporale Blutbehandlung charakteristischen Kenngrößen vor, wobei jeweils in Abhängigkeit von der einen oder vorzugsweise von einer der mehreren charakteristischen Kenngrößen eine bestimmte Blutflussrate ermittelt wird.

Hierauf wird aus einer Mehrzahl von Blutflussraten, welche auf Basis der charakteristischen Kenngrößen ermittelt worden sind, eine Blutflussrate ausgewählt, die für die aktuelle Behandlung dann vorgegeben wird. Die Auswahl der einen Blutflussrate erfolgt unter Anwendung eines (Auswahl-) Algorithmus, der in einer vorrichtungsinternen Software / Hardware implementiert ist. Der Algorithmus erlaubt eine automatische Auswahl der einen Blutflussrate.

Der Erfindung liegt angesichts dieses bekannten Stands der Technik die Aufgabe zugrunde, eine extrakorporale Blutbehandlungs-/-reinigungsmaschine, vorzugsweise Dialysemaschine, zu schaffen, mit der sich ein optimaler Blutfluss (für eine im wesentlichen maximale Reinigungsleistung) bei einer Dialysebehandlung einstellen lässt. Ein Ziel ist es dabei, den Blutfluss so einstellen zu können, dass eine Rezirkulation beispielsweise in einem Patienten-Shunt verringert oder vermieden wird. Ein weiteres Ziel ist es, das Verfahren die Vorrichtung möglichst einfach zu gestalten.

Die Aufgabe wird gelöst durch eine extrakorporale Blutbehandlungsmaschine (Blutreinigungsmaschine oder Dialysemaschine) nach Anspruch 1. Ausführungsformen der vorliegenden Erfindung sind Gegenstand der Unteransprüche.

Die Erfindung beruht zusammenfassend auf dem allgemeinen Vorgang, wonach der Blutfluss durch den Dialysator mit einer vorgegebenen Rate (d.h. innerhalb einer bestimmten (Prozess-) Zeit t ausgehend von einem vorbestimmten Startwert auf einen vorbestimmten Zielwert) vorzugsweise linear gesteigert wird, während dessen kontinuierlich oder getaktet mittels geeigneter Sensoren der aktuelle venöse und/oder arterielle Druck im extrakorporealen Blutkreislauf sowie dialyseseitige Merkmale/Eigenschaften (insbesondere der aktuelle Grad oder Menge an urämischen Toxinen) in einer verbrauchten Reinigungsflüssigkeit (Dialysierflüssigkeit) gemessen werden. Aus diesen konkreten Messwerten lässt sich dann der für die aktuelle Behandlung (individuell) vorteilhafteste Blutfluss vorzugsweise durch einen Vergleich der erfassten Messwerte mit vorab eingestellten oder bereits implementierten (standardisierten) Sollwerten bestimmen/auffinden.

Dabei ist die Erfassung/Detektion insbesondere der genannten dialyseseitigen Merkmale/Eigenschaften mit einer Totzeit $\Delta t$ (tatsächlich auftretende Verzögerungszeit zwischen der vorgenommenen Ratenänderung und dem an den Sensoren messbaren Resultat hieraus) verknüpft, die sich im Wesentlichen aus dem Abstand zwischen Dialysator und Sensor in Schlauchlängsrichtung sowie der (durchschnittlichen) Strömungsgeschwindigkeit der Reinigungsflüssigkeit ergibt. Diese Totzeit $\Delta t$ wird in die Bestimmungsroutine mit einbezogen bzw. berücksichtigt werden, um die endgültige Entscheidung bezüglich der optimalen Blutflusseinstellung zu treffen.

Zum Einstellen eines Blutflusses für eine im Wesentlichen optimale Reinigungsleistung können in der extrakorporalen Blutbehandlungs-/-reinigungsmaschine, vorzugsweise Dialysemaschine, die folgenden Schritte ausgeführt werden:

a) Vorgeben eines Blutflusszielwertes, Qb_ziel, vorzugsweise über eine Kommunikationseinrichtung,

b) Verändern eines (vorgegebenen/vorgebbaren) anfänglichen Blutflusses, Qb_start (unterschiedlich zu Qb_ziel), mit einer vorgegebenen/vorgebbaren Blutflussänderungsrate und damit über eine vorbestimmte maximale Blutflussveränderungszeit in Richtung hin zum Blutflusszielwert, Qb_ziel beispielsweise durch eine Blutpumpen/Steuereinheit,

c) Vergleichen eines gemessenen aktuellen venösen Drucks PV, mit einem (vorgegebenen oder ausgewählten) venösen Druckschwellenwert; eines gemessenen aktuellen arteriellen Drucks PA, mit einem (vorgegebenen oder ausgewählten) arteriellen Druckschwellenwert; und eines gemessenen aktuellen Blutflusses Qb, mit dem Blutflusszielwert, Qb_ziel durch eine Vergleichereinheit,

d) Erfassen wenigstens eines aktuellen Dialysierflüssigkeitsparameters/-merkmals/- eigenschaft (Grad/Menge an in einer verbrauchten Dialysierflüssigkeit enthaltenen urämischen Toxinen z.B. über UV-Absorbtion/Absorbanz) mit einer Zeitverzögerung/Totzeit $\Delta t$ bezüglich des Zeitpunkts des zugehörigen gemessenen Blutflusses (in Abhängigkeit von der Strömungsgeschwindigkeit der Dialysierflüssigkeit sowie des Strömungsabstands der Erfassungsstelle zum Dialysator) durch eine Erfassungseinrichtung und Ermitteln durch eine Ermittlungseinrichtung, ob der erfasste aktuelle Dialysierflüssigkeitsparameter einen Dialysierflüssigkeit-Parameterschwellenwert annähert/erreicht (Ermittlung des Auftretens eines Parameterextrems),

e) Abspeichern eines Blutflussoptimalwertes, Qb_optimum in einem Blutflussoptimalwertspeicher, in Abhängigkeit von jenem Blutfluss oder jener Blutflussrate, bei dem/der in Schritt d) der Dialysierflüssigkeit-Parameterschwellenwert (Parameterextrem) tatsächlich erreicht worden ist, oder bei dem in Schritt c) der venöse Druckschwellenwert PV bzw. der arterielle Druckschwellenwert PA erreicht worden ist und in Schritt d) der Dialysierflüssigkeit-Parameterschwellenwert noch nicht erreicht worden ist, wobei der Schritt d) um eine vorbestimmte Wartezeit tx ab Erreichen des venösen Druckschwellenwerts PV bzw. des arteriellen Druckschwellenwerts PA fortgeführt wird ,

f) Rückspringen zu Schritt b) vorzugsweise durch eine Rücksprungeinrichtung andernfalls.

Je nach Abstand der Erfassungsstelle zum Dialysator (in Strömungsrichtung der verbrauchten Dialysierflüssigkeit gesehen) sowie der Strömungsgeschwindigkeit der Dialysierflüssigkeit kann die Wartezeit null sein, wenn die Totzeit $\Delta t$ quasi null ist, oder vorzugsweise größer/gleich der Totzeit $\Delta t$ sein. Wenn in diesem Fall einer der Druckschwellenwerte PV, AV während der Blutflussveränderungszeit t bei einem aktuellen Blutfluss erreicht wird, würde dann nur noch für die Zeit tx abgewartet werden, ob sich dialyseseitig ein Parameterextrem (nachträglich) zeigt. Falls ja, würde der betreffende tatsächliche Blutfluss (kleiner dem aktuellen Blutfluss) bestimmt werden. Andernfalls wäre der aktuelle Blutfluss der Optimale.

An dieser Stelle sei noch erwähnt, dass der Blutflussoptimalwert auch geringfügig kleiner sein kann als der aktuelle/tatsächliche gemessene Blutfluss, für welchen einer der Druckschwellenwerte PV, AV oder das Parameterextrem erreicht worden ist.

Ferner sei darauf hingewiesen, dass die Wartezeit tx nicht unbedingt der Totzeit $\Delta t$ entsprechen muss. Insbesondere gilt: Wartezeit tx größer/gleich Totzeit $\Delta t$. Vorzugsweise gilt: Wartezeit tx = x * $\Delta t$ (mit x größer/gleich 1).

Die Blutreinigungsmaschine kann entweder

- bei dem eingestellten maximalen Blutfluss (1. Kriterium) oder
- ca. bei dem Blutfluss, in welchem der maximal zulässige arterielle Druck/venöse Druck (2. Kriterium) erreicht wird oder
- ca. bei dem Blutfluss, in welchem ein Parameterextrem (3. Kriterium) auftritt/auftrat, betrieben wird (entsprechend dem Kriterium, das zuerst erfüllt ist), auch dann, wenn sich das Parameterextrem (3. Kriterium) erst zeitlich verzögert zu den anderen Kriterien einstellt und daher ein ggf. bereits abgespeicherter (vorläufiger) Blutflussoptimalwert Qb_optimum (resultierend aus dem 1. oder 2. Kriterium) in jenen Blutflusswert reduziert wird, bei dem das (erst nachträglich) ermittelte Parameterextrem (3. Kriterium) aufgetreten ist.

Die Blutbehandlungsmaschine kann als weiteres Merkmal oder als Kombination weiterer Merkmale, soweit dies technisch möglich und sinnvoll ist, aufweisen, dass

- Schritt d) im Verlauf der vorgegebenen Wartezeit tx (theoretisch angenommener Wert, welcher der tatsächlichen Verzögerungszeit entsprechen kann oder diese zumindest annähert) nach Schritt c) kontinuierlich oder getaktet ausgeführt wird, wenn in Schritt c) ein Schwellenwert erreicht wird;
- die Blutflussänderungsrate von einem vorgegebenen/eingebbaren Blutflussanfangswert Qb_start, dem vorgegebenen/eingebbaren Blutflusszielwert Qb_ziel, und ggf. der vorgegebenen Blutflussänderungsdauer t, abhängig eingestellt wird;
- der vorgegebene Blutflussanfangswert, Qb_start, 50ml/min und vorzugsweise der vorgegebene Blutflusszielwert Qb_ziel, max. 600ml/min beträgt;
- der Blutflusszielwert Qb_ziel, als Defaultwert in einer Steuereinrichtung abgespeichert ist, über eine Kommunikationseinrichtung eingegeben wird, von einer Patientenkarte eingelesen wird oder von einem Server übertragen wird;
- die Totzeit $\Delta t$ in Abhängigkeit von der Blutflussänderungsrate, dem Blutflusszielwert Qb_ziel, einem Dialysierflüssigkeitsfluss Qd, und Parametern der extrakorporalen Blutbehandlungs-/-reinigungsmaschine, vorzugsweise Dialysemaschine (siehe vorzugsweise Parameterdefinition gemäß nachfolgender Figurenbeschreibung) vorgegeben ist;
- die Parameter der extrakorporalen Blutbehandlungsmaschine mit Hilfe der Kommunikationseinrichtung eingegeben werden, von einem Barcode eingelesen werden oder von einem Server, der Behandlungseinstellungen für Patienten enthält, geladen werden;

- die Totzeit ∆t, und der Blutflusszielwert, Qb_ziel, als Wertepaare in Abhängigkeit von Parametern der extrakorporalen Blutbehandlungsmaschine in einer Wertetabelle abgespeichert sind.

Die extrakorporale Blutbehandlungs-/-reinigungsmaschine, vorzugsweise Dialysemaschine, hat vorzugsweise zur Durchführung des vorstehend beschriebenen Steuerungsverfahrens die folgenden Merkmale:

- einen Dialysator zum Blutreinigen,
- wenigstens eine Blutpumpe zum Erzeugen eines extrakorporalen Blutflusses zwischen einem Patienten und dem Dialysator,
- wenigstens eine Dialysierflüssigkeitspumpe zum Versorgen des Dialysators mit einer Dialysierflüssigkeit,
- wenigstens einen venösen Blutdruckaufnehmer stromab (unterhalb) des Dialysators,
- wenigstens einen arteriellen Blutdruckaufnehmer stromauf (oberhalb) des Dialysators,
- wenigstens einen Dialysierflüssigkeitssensor zum Erfassen wenigstens eines Dialysierflüssigkeitsparameters (z.B. UV-Absorption/-Absorbanz) unterhalb (stromab) des Dialysators in einem bestimmten Strömungsabstand zum Dialysator,
- optional wenigstens einen Blutflusssensor zum Erfassen des extrakorporalen Blutflusses falls dieser nicht mittels der Pumpe gezielt einstellbar ist,
- optional wenigstens einen Dialysierflüssigkeit-Flusssensor zum Erfassen eines Dialysierflüssigkeitsflusses falls dieser nicht mittels der Pumpe gezielt einstellbar ist.

Die Blutbehandlungsmaschine, wie Dialysevorrichtung, kann eines oder mehrere der folgenden Merkmale aufweisen:

- eine Kommunikationseinrichtung zum Vorgeben eines extrakorporalen Blutflusszielwertes Qb_ziel, und optional eines extrakorporalen Blutflussstartwertes Qb_start und
- eine Regel-/Steuereinrichtung zum Einstellen/Justieren eines extrakorporalen Blutflusswertes des Blutflusses (in Abhängigkeit von dem Blutflusszielwert Qb_ziel), die aufweist:
- ein Steuermittel zum Verändern (Erhöhen/ggf. Verringern) des extrakorporalen Blutflusses Qb, mit einer vorgegebenen oder ausgewählten Blutflussänderungsrate,
- einen Vergleicher zum Vergleichen eines (aktuell gemessenen) venösen Drucks PV mit einem vorgegebenen oder ausgewählten venösen Druckschwellenwert, eines (aktuell gemessenen) arteriellen Drucks PA mit einem vorgegebenen oder ausgewählten arteriellen Druckschwellenwert und des aktuellen Blutflusses Qb, mit dem Blutflusszielwert, Qb_ziel,
- eine Erfassungseinrichtung zum Erfassen wenigstens eines aktuellen Dialysierflüssigkeitsparameters/- merkmals/- eigenschaft (Grad/Menge an in einer verbrauchten Dialysierflüssigkeit enthaltenen urämischen Toxinen z.B. über UV-Absorbtion/Absorbanz),
- eine Ermittlungseinrichtung zum Ermitteln, ob der erfasste aktuelle Dialysierflüssigkeitsparameter einen Dialysierflüssigkeit-Parameterschwellenwert annähert/erreicht hat (Ermittlung des Auftretens eines Parameterextrems), und/oder
- einen Blutflussoptimalwertspeicher zum Abspeichern jenes Blutflusses als Blutflussoptimalwert Qb_optimum, bei dem der Dialysierflüssigkeit-Parameterschwellenwert (Parameterextrem) erreicht worden ist, oder zum vorläufigen Abspeichern jenes Blutflusses als Blutflussoptimalwert Qb_optimum, bei dem der venöse Druckschwellenwert bzw. der arterielle Druckschwellenwert erreicht worden ist (und der Dialysierflüssigkeit-Parameterschwellenwert noch nicht erreicht worden ist) oder zum Abspeichern des extrakorporalen Blutflusszielwertes Qb_ziel als Blutflussoptimalwert Qb_optimum, falls weder der Parameterschwellenwert noch der venöse Druckschwellenwert bzw. der arterielle Druckschwellenwert (vorher) erreicht wurden.
- Die Regel-/Steuereinrichtung kann zudem erfindungsgemäß dazu ausgelegt sein, zumindest die Erfassungs- und Ermittlungseinrichtung auch dann noch weiter über eine Wartezeit tx zu betreiben, selbst wenn der Vergleicher das Erreichen des extrakorporalen Blutflusszielwerts Qb_ziel oder des ausgewählten venösen / arteriellen Druckschwellenwerts zu einem bestimmten Zeitpunk, in welchem die Wartezeit tx gestartet wird, erkannt hat (und damit der Anstieg des Blutflusses gestoppt wurde), um nachträglich den bereits abgespeicherten vorläufigen Blutflussoptimalwert Qb_optimum auf jenen Blutflusswert zu justieren/verändern (wieder zu reduzieren), bei dem ggf. ein Paramterextrem zeitlich um eine Verzögerungszeit/Totzeit ∆t verzögert, ermittelt wird. Für das Verhältnis zwischen Wartezeit tx und Totzeit ∆t gelten die vorstehenden Definitionen.

Bevorzugte Ausführungsformen der erfindungsgemäßen Blutbehandlungsmaschine insbesondere Dialysemaschine weisen als weiteres Merkmal oder als Kombination weiterer Merkmale, soweit dies technisch möglich und sinnvoll ist, auf, dass

- die Steuereinrichtung eine Zeitverlängerungseinheit hat zum Verlängern des Ermittelns durch die Ermittlungseinrichtung gegenüber dem Vergleichen durch den Vergleicher um die vorgegebene oder einstellbare Wartezeit, tx, insbesondere wenn vom Vergleicher das Erreichen/Überschreiten eines Schwellenwerts erkannt wird/wurde;
- das Steuermittel vorzugsweise in Form einer Blutpumpensteuereinheit die Blutflussänderungsrate in Abhängigkeit von einem vorgegebenen Blutflussanfangswert Qb_start dem Blutflusszielwert, Qb_ziel und einer vorgegebenen Blutflussänderungsdauer t berechnet/auswählt;
- das Steuermittel, vorzugsweise die Blutpumpensteuereinheit den vorgegebenen Blutflussanfangswert Qb_start auf 50ml/min festsetzt und optional den Blutflusszielwert Qb_ziel auf > 50 bis max. 600ml/min festsetzt;
- das Steuermittel, vorzugsweise die Blutpumpensteuereinheit den Blutflusszielwert Qb_ziel als Defaultwert in einer Steuereinrichtung ausliest, von einer Kommunikationseinrichtung einliest, von einer Patientenkarte einliest oder von einem Server einliest;
- die Zeitverlängerungseinheit die Wartezeit tx in Abhängigkeit von der Totzeit $\Delta t$ bestimmt, die sich aus der Blutflussänderungsrate, dem Blutflusszielwert Qb_ziel einem Dialysierflüssigkeitsfluss Qd und Parametern der Blutbehandlungsmaschine / Dialysemaschine ergibt;
- die Zeitverlängerungseinheit die Wartezeit tx aus einer Wertetabelle einliest, wobei in der Wertetabelle die Wartezeit tx oder die Totzeit $\Delta t$ und der Blutflusszielwert Qb_ziel als Wertepaare in Abhängigkeit von Parametern der Blutbehandlungsmaschine / Dialysemaschine abgespeichert sind.

Die Erfindung hat u. a. die folgenden Vorteile:

Dem Arzt wird eine bessere Einschätzung des zu wählenden Blutflusses für eine Behandlung ermöglicht. Es wird ein schnelles Online-Verfahren zur Blutflussfindung ermöglicht. Das Anlegen und Regeln des Blutflusses beispielsweise bei einer Dialysebehandlung kann mit dem erfindungsgemäßen Verfahren automatisch ablaufen.
Der Blutfluss wird zur Maximierung der dialysierflüssigkeitsseitigen Toxinmenge unmittelbar (online) angepasst. Dazu wird die dialysierflüssigkeitsseitige Toxinmenge mittels eines optischen Sensores (online) überwacht. Ebenfalls wird (online) der arterielle und venöse Druck mittels Drucksensoren an der Blutbehandlungsmaschine / Dialysemaschine überwacht, um die Sicherheit zu gewährleisten.

Weitere Merkmale und Vorteile der Erfindung ergeben sich aus der folgenden Beschreibung von bevorzugten Ausführungsformen, bei der Bezug genommen wird auf die beigefügte Zeichnung.

**Figurenbeschreibung**

Fig. 1 zeigt schematisch eine Ausführungsform der erfindungsgemäßen Blutbehandlungsmaschine / Dialysemaschine.

Fig. 2 zeigt zur Erläuterung den Verlauf der Clearance gegenüber dem Blutfluss mit und ohne Störeffekt.

Fig. 3 zeigt zur Erläuterung den Verlauf der Extinktion in der Dialysierflüssigkeit gegenüber dem Blutfluss mit und ohne Störeffekt.

Fig. 4 zeigt eine Ausführungsform eines (Maschinensteuerungs-) Verfahrens zum Einstellen des aktuellen Blutflusses bei einer Dialyse.

Fig. 5A und 5B zeigen zur Erläuterung jeweils schematisch den Weg einer möglichen Störung in einem Dialysesystem.

Fig. 6A und 6B zeigen zur Erläuterung jeweils schematisch die Bestimmung eines Extremalwerts im Intensitätssignal eines Dialysierflüssigkeitssensors in einem Dialysesystem.

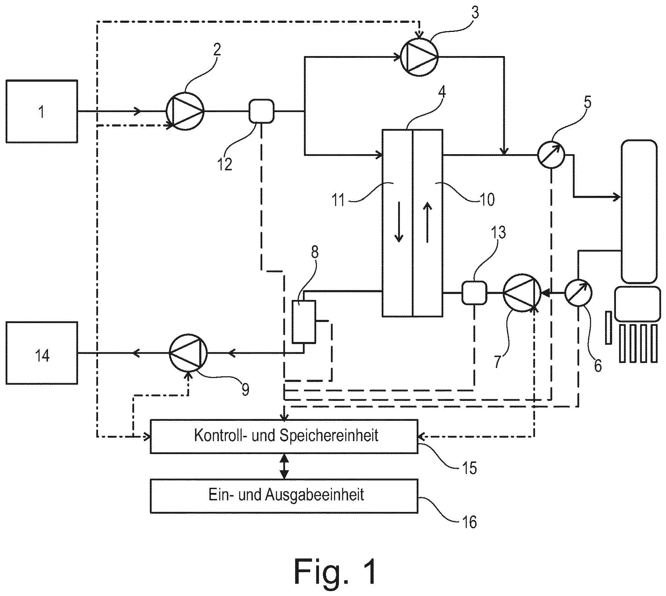

Fig. 1 zeigt den schematischen Flussplan einer extrakorporalen Blutbehandlungs-/- reinigungsmaschine / Dialysemaschine mit einer Steuereinrichtung 15 in Verbindung mit einer Kommunikationseinrichtung 16. Eine Blutpumpe 7 entnimmt aus einem Patientenkörper Blut über einen Patientenzugang (Shunt-Punktierungsstelle), der in Fig. 1 rechts schematisch als Patienten-Unterarm angedeutet ist. Die aktuelle Blutpumpenrate kann optional mit einem Flusssensor 13 auf der extrakorporalen Blutseite zusätzlich bestimmt werden. Ein arterieller Druckaufnehmer 6 überwacht den aktuellen Unterdruck auf der Saugseite der Blutpumpe 7.

Die Blutpumpe 7 fördert das Blut des Patienten durch einen Dialysator 4 hindurch, wobei urämische Toxine von einer Blutseite 10 auf eine Dialysierflüssigkeitsseite 11 des Dialysators 4 durch eine semipermeable Membran (nicht dargestellt) hindurch übertreten können. Das dadurch gereinigte Blut wird dann zum Patienten zurückgeführt. Ein venöser Druckaufnehmer 5 überwacht den venösen Druck innerhalb des extrakorporalen Blutkreises stromab zum Dialysator 4.

Dialysierflüssigkeitspumpen 2 und 9 erzeugen den Dialysierflüssigkeitsdurchfluss durch den Dialysator 4 auf dessen Dialysierflüssigkeitsseite 11. Dadurch werden die im Dialysator 4 auf die Dialysierflüssigkeitsseite 11 übergetretenen urämischen Toxine an einem optischen Sensor 8 vorbeigeführt, der dem Dialysator 4 dialysierflüssigkeitsseitig vorzugsweise unmittelbar nachgeschaltet ist. Die Menge an urämischen Toxinen, welche auf die Dialysierflüssigkeitsseite 11 übergetreten sind, kann mittels des optischen Sensors (UV-Sensor) 8 vermessen werden. Die Intensität I bzw. Absorbanz A in der verbrauchten Dialysierflüssigkeit ist dabei ein Maß für die aktuelle Reinigungsleistung der Blutbehandlungsmaschine / Dialysemaschine.

Die verbrauchte Dialysierflüssigkeit durchströmt den optischen Sensor 8 mit einem definierten Dialysierflüssigkeitsfluss Qd, der mit einem Flusssensor 12 auf der Dialysierflüssigkeitsseite gemessen wird und der vorliegend dem Dialysator 4 vorzugsweise unmittelbar vorgeschaltet ist.

Der hydraulisch/fluidische Aufbau der vorstehend beschriebenen Blutbehandlungsmaschine / Dialysemaschine entspricht weitgehend dem Stand der Technik und ist damit grundsätzlich bekannt. Auch der Betrieb der Blutbehandlungsmaschine / Dialysemaschine ist aus dem Stand der Technik per se bekannt, sodass die detaillierte Beschreibung der einzelnen, am Dialysator 4 sich einstellenden Vorgänge an dieser Stelle entfallen kann.

Die Reinigungsleistung des Dialysators 4 soll in der Regel so hoch wie möglich sein. Da der extrakorporale Blutfluss, neben dem Dialysierflüssigkeitsfluss und der Qualität des Dialysators 4 eine der Stellgrößen zur Maximierung der Reinigungsleistung der Blutbehandlungsmaschine / Dialysemaschine darstellt, dient die vorliegend beschriebene Vorrichtung und Verfahren gemäß der Erfindung der Maximierung der Reinigungsleistung durch Anpassung des extrakorporalen Blutflusses.

Hierfür wird mittels des optischen Sensors 8 der dialysierflüssigkeitsseitige Ausgleichsvorgang während und nach Abschluss einer definierten Blutflussänderung mit Hilfe des optischen Sensors 8 analysiert. Es handelt sich daher prinzipiell um eine Transientenmessung, die sich sowohl für Hämodialyse (HD), Hämodiafiltration (HDF), Hämofiltration (HF) als auch "Single needle cross over" (SNCO) für die Optimierung des Blutflusses eignet.

Fig. 1 zeigt, welche Einheiten der Maschine für die Umsetzung der erfindungsgemäßen Blutflussregelung notwendig sind und in welcher Weise sie kommunizieren. Eine elektronische Kommunikationseinrichtung 16 dient dem Benutzer einerseits zur Anzeige und andererseits zur Eingabe von Behandlungsparametern (entsprechen den Parametern der Blutbehandlungsmaschine), wie z.B. Blutfluss (Start- und/oder Ziel-Blutfluss), Dialysierflüssigkeitsfluss und/oder Druckgrenzen im Bereich des Blutausgangs und Bluteingangs aus und in den Patienten. Dies geschieht beispielsweise über eine graphische Benutzeroberfläche der Maschine, mittels Patientenkarte oder via Datenübertragung der patientenindividuellen Behandlungseinstellungen von einem externen Patientendaten-Server.

Diese Eingaben werden von der Steuereinrichtung 15 verwendet, um die Pumpen 2, 9, 7 und/oder nicht weiter gezeigte Ventile zu steuern und/oder zu regeln. Dafür wird z. T. auf Informationen zurückgegriffen, welche von den (Blut-)Drucksensoren 5, 6, dem Flusssensor 12 auf der Dialysierflüssigkeitsseite, dem Flusssensor 13 auf der Blutseite und dem optischen Sensor 8 zur Verfügung gestellt werden. Die Steuereinrichtung 15 dient außerdem der Vorverarbeitung der Daten, die die Sensoren liefern. Die Vorverarbeitung beinhaltet z. B. Filtern und Glätten der Daten (-signale) bzw. die Extraktion von Parametern. Die Steuereinrichtung 15 dient darüber hinaus dem Abspeichern aller Behandlungsparameter (Maschinenparameter), aber auch als Speicher für alle jene Informationen, die notwendig sind, um das Verfahren zur optimierten Blutflusseinstellung anwenden zu können. Ein Beispiel hierfür sind dialysatorspezifische Daten, wie z.B. das blutseitige Volumen (Vb) und das dialysierflüssigkeitsseitige Volumen (Vd) des Dialysators 4, aber auch alle sonstigen Kennlinien und Kennfelder (z. B. Dialysatorclearance, etc.) gehören dazu.

Der Dialysator 4 und das extrakorporale Schlauchsystem / Blutleitungssystem müssen für die Anwendung einwandfrei identifiziert sein. Dies kann beispielsweise mit Hilfe der Kommunikationseinrichtung 16 erfolgen, z. B. durch die Eingabe des. Dialysatormodels durch den Benutzer oder das Einlesen eines am Dialysator 4 angebrachten Barcodes. Alternativ wäre es noch möglich, durch automatisches Pegelsetzen in den Dialysatorkammern ein definiertes Volumen zu erzeugen.

Die Kommunikation zwischen allen Modulen wie Steuereinrichtung 15, Kommunikationseinrichtung 16, die einzelnen Aktoren/Pumpen 2, 9, 7, etc. kann unidirektional oder bidirektional erfolgen.

Die Intensität bzw. Absorbanz der verbrauchten Dialysierflüssigkeit ist ein direktes Maß für die Menge an urämischen Toxinen, die von Blut- nach der Dialysierflüssigkeitsseite übertreten. Häufig wird dabei von der Annahme ausgegangen, dass eine Erhöhung des extrakorporalen Blutflusses (Qb) auch immer mit einer Erhöhung der effektiven Clearance (Ce) einhergeht. Eine Erhöhung der Clearance hätte demnach eine größere Menge an urämischen Toxinen auf Dialysierfllüssigkeitsseite zur Folge und könnte über eine höhere Absorbanz der verbrauchten Dialysierflüsigkeit erfasst werden. Diese Annahme besitzt allerdings nur dann Gültigkeit, wenn keine Komplikationen auftreten, die die Menge an urämischen Toxinen bereits am Gefäßzugang des Patienten verringern. Eine solche Komplikation, wie z.B. eine lokale Rezirkulation oder Shunt-Rezirkulation, hat zur Folge, dass die Menge an urämischen Toxinen, die den Weg auf die Dialysierflüssigkeitsseite findet, trotz steigenden Blutflusses nicht oder nicht in gleichem Maß zunimmt, sondern beispielsweise gleich bleibt, weniger stark zunimmt oder sogar sinkt. Der Zusammenhang zwischen Clearance Ce und Rezirkulation kann dabei wie folgt beschrieben werden:

$$Ce = (1 - R) Cd / (1 - R (1 - Cd / Qb)) \qquad (1)$$

mit Ce als der effektiven Clearance "aus Patientensicht" (entspricht nicht notwendiger weise der Dialysator-Clearance), R als Rezirkulation und Cd als Clearance des Dialysators. Das bedeutet, dass die effektive Clearance beim Auftreten von Rezirkulation / Shunt-Rezirkulation deutlich hinter der reinen Dialysator-Clearance zurückbleibt, die für R = 0 gleich der effektiven Clearance wäre.

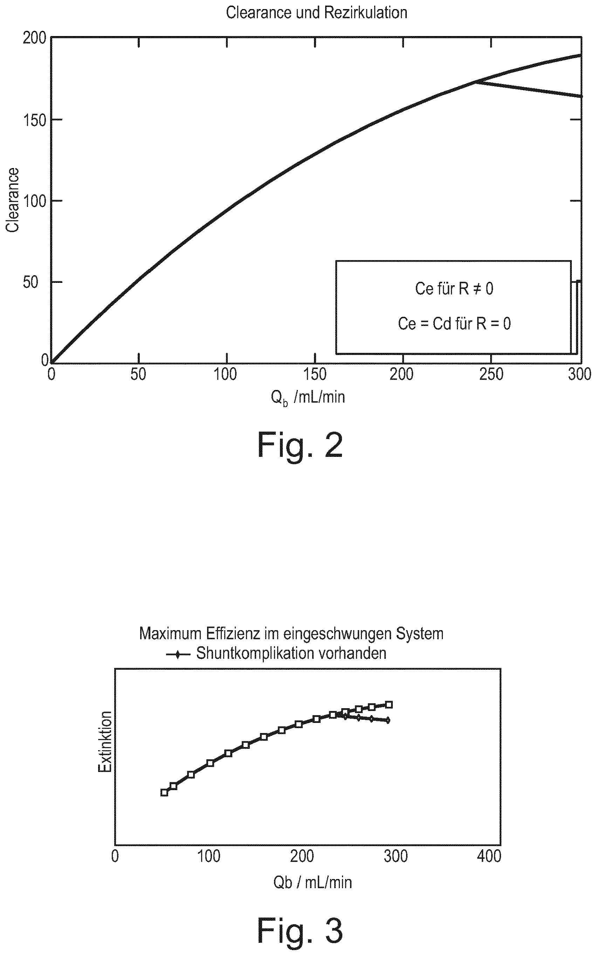

Fig. 2 zeigt zur Erläuterung ein Beispiel für die Verringerung der effektiven Clearance Ce eines Dialysators unter der Annahme, dass bei einem Blutfluss von 300ml/min eine Rezirkulation von 20% erfolgt.

In Fig. 2 ist die Clearance als eine Funktion des Blutflusses Qb nach Gleichung (1) dargestellt. Steigt der Blutfluss Qb, steigt auch die effektive Clearance. Die Clearance hat einen nichtlinearen und für den rezirkulationsfreien Fall einen monoton ansteigenden Verlauf. Kommt es aber zu einer Rezirkulation am Patientenzugang, dann verringern die Rezirkulationseffekte die effektive Clearance Ce, was in Fig. 2 als die bei hohen Qb-Werten nach unten von der theoretischen/idealen effektiven Clearance abzweigende/abweichende Linie dargestellt ist.

Fig. 3 zeigt zur Erläuterung ein Beispiel für eine sog. steady-state-Absorbanz, gemessen bei ca. 285nm hinter dem Dialysator 4. Dargestellt ist die dialysierflüssigkeitsseitige Absorbanz über dem Blutfluss. Bei hohen Qb-Werten ist als fallende Linie die Verringerung der Absorbanz durch Rezirkulationseffekte aufgetragen.

Die Absorbanz ist äquivalent zur Menge an aus dem Blut entfernten urämischen Toxinen. Die Reinigungsleistung ist daher maximal, wenn die Menge an Toxinen auf der Dialysierflüssigkeitsseite für definierte Qb, Qd maximal ist. Damit ist die Reinigungsleitung auch maximal, wenn die Absorbanz maximal bzw. die Intensität am Messkanal des Sensors minimal ist.

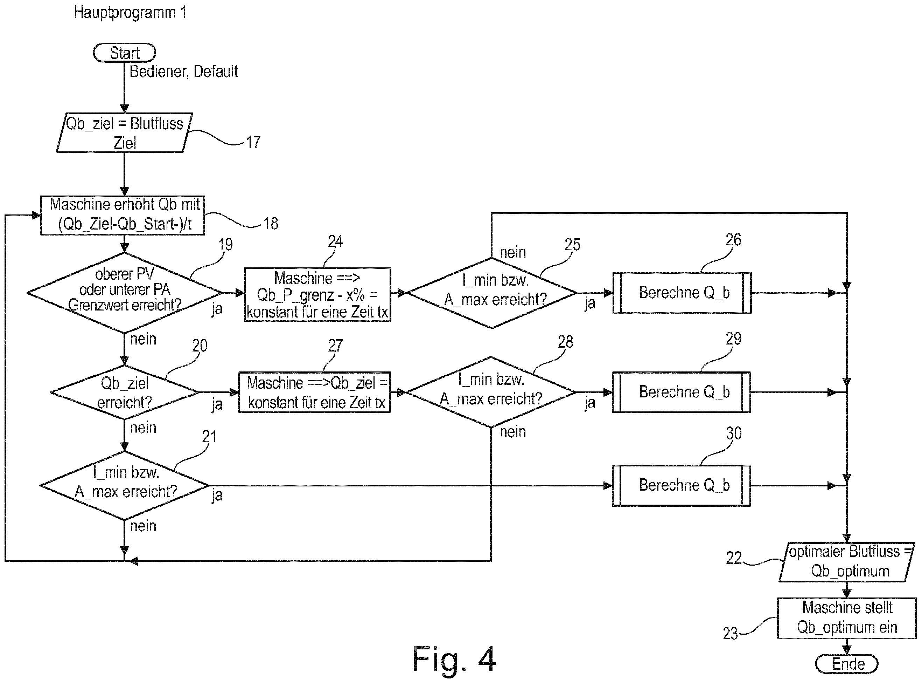

Fig. 4 zeigt eine Ausführungsform eines Verfahrens. Bei dem Verfahren wird durch die Kombination von einer, vorzugsweise linearen, Blutflussänderung, der Analyse der Drucksignale und der optischen Messung der für die Behandlung vorteilhafteste Blutfluss gefunden.

Die Ausführungsform des (Maschinensteuerungs-) Verfahrens nach Fig. 4 weist die Schritte ausgehend von einem ausgewählten oder voreingestellten Blutfluss-Startwert Qb_start

- 17 Vorgeben eines Blutflusszielwerts Qb_ziel,
- 18 Verändern des Blutflusses Qb mit einer vorgegebenen, ggf. linearen Blutflussänderungsrate vorzugsweise durch eine Blutpumpensteuereinheit oder ein Stromregelventil, oder dergleichen,
- 19 Vergleichen eines (blutseitigen) venösen Drucks PV mit einem ausgewählten oder vorgegebenen venösen Druckschwellenwert und eines (blutseitigen) arteriellen Drucks PA mit einem ausgewählten oder vorgegebenen arteriellen Druckschwellenwert,
- 20 Vergleichen des aktuellen Blutflusses Qb mit dem Blutflusszielwert Qb_ziel, vorzugsweise durch eine Vergleichereinheit,
- 21 Erfassen wenigstens eines Dialysierflüssigkeitsparameters (z.B. Absorbanz) sowie vorzugsweise Nachverfolgen des Parameterverlaufs durch eine Erfassungseinrichtung und Ermitteln eines möglichen Auftretens eines Parameterextrems durch eine Ermittlungseinrichtung,
- 22 Abspeichern eines Blutflussoptimalwertes Qb_optimum basierend auf den Ergebnissen in den Schritten 19 bis 21,
- 23 Betreiben der Maschine mit dem Blutflussoptimalwertes Qb_optimum bis auf weiteres,
- 24 Betreiben der Maschine mit dem Blutflusswert Qb_P_grenz - x% während einer vorgegebenen Wartezeit tx,
- 25 Nachträgliches/fortlaufendes Ermitteln eines möglichen Auftretens eines Parameterextrems durch die Ermittlungseinrichtung zumindest in der Wartezeit tx oder darüber hinaus,
- 26 Neuberechnen/Nachjustieren des Blutflusswertes Q_b (im Fall des nachträglich ermittelten Auftretens eines Parameterextrems)
- 27 Betreiben der Maschine mit dem Blutflusszielwert Qb_ziel während einer vorgegebenen Wartezeit tx,
- 28 Nachträgliches/fortlaufendes Ermitteln eines möglichen Auftretens eines Parameterextrems durch die Ermittlungseinrichtung zumindest in der Wartezeit tx oder darüber hinaus,
- 29 Neuberechnen/Nachjustieren des Blutflusswertes Q_b (im Fall des nachträglich ermittelten Auftretens eines Parameterextrems) und

- 30 Neuberechnen/Nachjustieren des Blutflusswertes Q_b (im Fall des ermittelten Auftretens eines Parameterextrems).

Diese Schritte werden im folgenden einzeln erläutert.

In Schritt 17 wird ein Zielwert für den Blutfluss, Qb_ziel, eingegeben (z.B. >50-600ml/min). Der Blutflusszielwert, Qb_ziel, kann als Defaultwert in der Steuereinrichtung 15 abgespeichert sein, über die Kommunikationseinrichtung 16 eingegeben werden, von einer (nicht gezeigten) Patientenkarte eingelesen werden oder von einem (nicht gezeigten) Server übertragen werden.

In Schritt 18 wird innerhalb einer vorgegebenen Zeit t der aktuelle Blutfluss Qb von einem vorgegebenen Wert Qb_start (z.B. 50ml/min) auf den Wert Qb_ziel (kontinuierlich oder schrittweise mit einer bestimmten Steigerungsrate) angehoben. Der vorgegebene Wert Qb_start beträgt z.B. 50ml/min und ist festgelegt, während der Wert Qb_ziel (z.B. 300ml/min) wie erwähnt vom Benutzer mittels der Kommunikationseinrichtung 16 vorgegeben oder von einer Patientenkarte oder einem Server übertragen werden kann.

Durch die Steuereinrichtung 15 werden kontinuierlich oder getaktet Parameter abgefragt, welche als Abbruch- bzw. Kontrollkriterien für die Blutflusssteigerung dienen. Diese Kriterien sind:

  i. Erreichen/Überschreiten eines der Druckwertgrenzen (oberer Grenzwert venöser Druck PV und/oder unterer Grenzwert arterieller Druck PA im extrakorporalen Blutkreis),
  ii. Auftreten eines Intensitätsminimums bzw. Absorbanzmaximums im Signal des optischen Sensors,
  iii. Erreichen/Überschreiten des angeforderten hohen Blutflussniveaus Qb_ziel.

In Schritt 19 wird folglich abgefragt, ob ein oberer Grenzwert für den venösen Druck, PV, bzw. ein unterer Grenzwert für den arteriellen Druck, PA, erreicht/überschritten wurde.

In Schritt 20 wird außerdem abgefragt, ob der Zielwert des Blutflusses, Qb_ziel, erreicht worden ist. Der Zielwert des Blutflusses, Qb_ziel, kann, wie oben erwähnt, von dem Benutzer manuell über die Kommunikationseinrichtung 16 eingestellt werden oder als Defaultwert vorgegeben sein, wobei der Defaultwert aus der Steuereinrichtung 15 geladen wird.

Desgleichen wird in Schritt 21 abgefragt, ob ein Intensitätsminimum I_min bzw. Absorbanzmaximum A_max im Signal des optischen Sensors 8 für die Konzentration urämischer Toxine in der Dialysierflüssigkeit unterhalb des Dialysators 4 identifiziert wurde.

Fig. 4 zeigt die weiteren Schritte des Verfahrens in Abhängigkeit davon, bei welcher der Abfragen 19 bis 21 eine Abzweigung des Ablaufs zuerst erreicht ist.

Wenn die Abfrage in Schritt 19 "Ja" ist, d.h. das Erreichen einer der beiden Grenzdrücke PV und PA erkannt wird, so wird ein Blutfluss (zunächst vorrübergehend) eingestellt, bei dem der Druck innerhalb der Druckgrenzen bleibt. Dieser Blutfluss wird dann für eine Wartezeit tx konstant gehalten. Diese Wartezeit tx entspricht in etwa einer zu erwartenden Verzögerungs- oder Totzeit, die verstreicht, bis ein am Dialysator 4 sich einstellendes Parameterextrem über die Dialyseabflussleitung vom Dialysator 4 zu dem (Absorbtions-) Sensor 8 braucht. Unabhängig davon wird optional das Signal des optischen Sensors 8 weiterhin permanent ausgewertet, bis der Ausgleichvorgang auf Dialysierflüssigkeitsseite vollständig abgeschlossen ist, d.h. bis sich ein stabiles Endniveau eingestellt hat und sich das Signal am optischen Sensor 8 nicht mehr ändert.

Wird von der Steuereinrichtung 15 kein Extremwert in Form eines Intensitätsminimums bzw. Absorbanzmaximums identifiziert, so ist der (zunächst vorläufig) konstant eingestellte Blutfluss auch der optimale Blutfluss für die Behandlung. Dies wird in Schritt 25 abgefragt, nach welchem bei negativem Ergebnis das Verfahren direkt mit dem Schritt 22 fortfährt. In Schritt 22 wird der optimale Blutfluss als Blutflussoptimalwert Qb_optimum in einem Blutflussoptimalwertspeicher abgespeichert, und die Maschine wird in Schritt 23 "endgültig" auf diesen Wert eingestellt. Das Verfahren ist damit abgeschlossen und wird beendet.

Wird dagegen in Schritt 25 von der Steuereinrichtung 15 ein Extremwert in Form eines Intensitätsminimums bzw. Absorbanzmaximums im Signal des optischen Sensors erkannt, so erfolgt die Berechnung des optimalen Blutflusses auf dieser Basis. Hierbei wird durch die Steuereinrichtung 15 mit Hilfe des Zeitpunktes, an dem das Extremum auftritt bzw. aufgetreten ist (Auftretenszeit), der Blutfluss berechnet (rekonstruiert). Dies erfolgt in Schritt 26, in welchem der Blutfluss dann als neuer Blutflusswert Q_b vorliegt.

Wenn in Schritt 21 festgestellt wird, dass ein Intensitätsmaximum I_min bzw. ein Absorbanzminimum A_max erreicht worden ist, so wird durch die Steuereinrichtung 15 beispielsweise bei der Auswertung des Transientensignals von dem optischen Sensor 8 das Auftreten eines Extremwertes (verzögert) detektiert. Auf dieser Basis wird durch die Steuereinrichtung 15 in Schritt 30 sowie den Schritten 22 und 23 dann der neue Blutflusswert Q_b und daraus der optimale Blutfluss Blutflussoptimalwert Qb_optimum berechnet.

Wenn in Schritt 20 festgestellt wird, dass der Blutflusszielwert Qb_ziel für den Blutfluss erreicht wurde (zunächst ohne dass ein Extremwert ermittelt worden ist), wird der Blutfluss in Schritt 27 für eine Wartezeit tx (vorläufig) konstant gehalten. Das Signal des optischen Sensors 8 wird dennoch weiterhin durch die Steuereinrichtung 15 ausgewertet.

Sollte nachträglich, d.h. nach der vorläufigen Einstellung des Blutflusses auf den Blutflusszielwert Qb_ziel (verzögert) vorzugsweise innerhalb der Wartezeit tx ein Parameterextrem doch noch ermittelt werden, wird in diesem Fall in Schritt 29 ein neuer Blutflusswert Q_b berechnet (entspricht in etwa jenem Blutfluss, der bei tatsächlichem Auftreten des Extrems geherrscht hat) und in Abhängigkeit von diesem wiederum der Blutflussoptimalwert Qb-optimum in Schritt 22 bestimmt sowie in Schritt 23 die Maschine bis auf weiteres mit diesem Wert betrieben, so dass das Verfahren damit endet. Konnte durch die Steuereinrichtung 15 vorzugsweise innerhalb der Wartezeit tx oder darüber hinaus kein Extremwert in Form eines Intensitätsminimums bzw. Absorbanzmaximums gefunden/ermittelt werden, so folgt in Schritt 28 ein Rücksprung zu Schritt 18, und der Blutfluss wird weiter erhöht, bis entweder in Schritt 19 der Venösschwellenwert PV oder der Arteriellschwellenwert PA erreicht wird oder in Schritt 21 der Dialysierflüssigkeitsschwellenwert I_min bzw. A_max erreicht wird. Alternativ hierzu könnte aber auch der behandelnde Arzt oder die betreuende Bedinerperson dazu aufgefordert werden, den Blutflusszielwert Qb_ziel neu vorzugeben.

Die Überwachung der Druckwerte PV, PA dient dabei dem Zweck, den zulässigen unteren arteriellen bzw. oberen venösen Druck nicht zu über- oder zu unterschreiten, und damit der Patientensicherheit. Beide Druckwerte werden durch das Zusammenspiel von Nadeln, Anstechsituation und Gefäßstatus des Patienten beeinflusst. Die Überwachung der Druckwerte erfolgt durch die Steuereinrichtung 15.

Der optische Sensor 8 auf Dialysierflüssigkeitsseite misst die Intensität bzw. die Absorbanz der verbrauchten Dialysierflüssigkeit in Anhängigkeit von den darin gelösten, ausgeschwemmten urämischen Toxinen. Die Steuereinrichtung 15 sucht einen Extrempunkt im Sensorsignal, welcher sich nur beim Auftreten von Komplikationen, z.B. einer lokalen Rezirkulation, wie Shunt-Rezirkulation, einstellen würde,

Die Signale der Drucksensoren 5, 6 und des optischen Sensors 8 sind Grundlage für die Aktionen, welche von der Steuereinrichtung 15 ausgelöst werden. Alle Sensordaten können durch die Steuereinrichtung 15 bearbeitet werden, z.B. geglättet werden oder durch einen Tiefpass gefiltert werden. Fig. 4 zeigt speziell für die Abfrage 20 "Qb_ziel erreicht?" und Abfrage 19 "PV oder PA erreicht?", dass anschließend nicht sofort auf den optimalen Blutfluss geschlossen werden kann. Die Ursache hierfür ist die folgende:

Die Werte zur Beurteilung der Abfrage 20 "Qb_ziel erreicht?" und der Abfrage 19 "PV oder PA erreicht?" sind sofort verfügbar. Dies gilt jedoch nicht in gleichem Maße für die Daten des optischen Sensors 8.

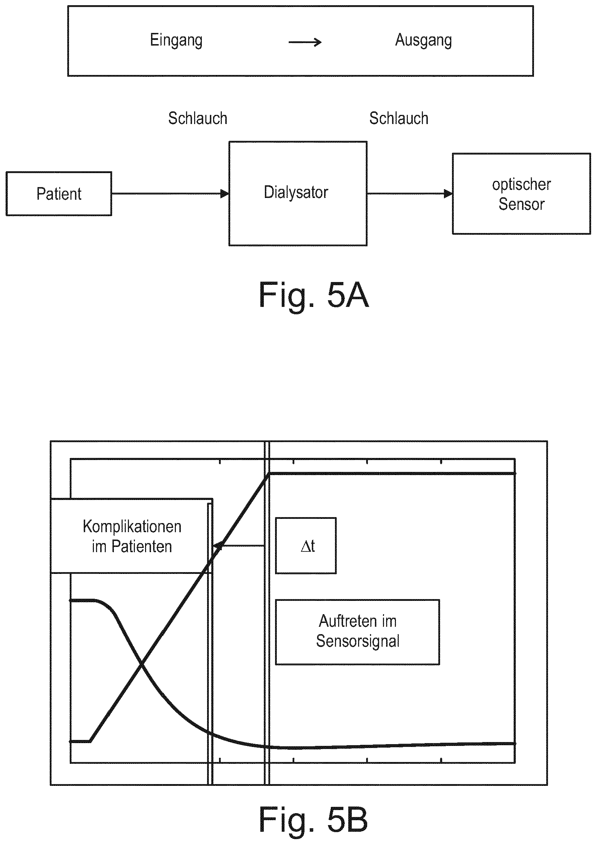

Die zeitliche Abfolge einer Druckänderung und der Reaktion am optischen Sensor 8 wird im folgenden anhand von Fig. 5A und 5B erläutert. In Fig. 5A ist die Richtung schematisch angedeutet, in welcher die Prozesse im folgenden erläutert werden. Der Patient befindet sich am Eingang des Systems. Er ist über ein blutseitiges Schlauchsystem mit dem Dialysator 4 verbunden. Der Dialysator 4 ist seinerseits über ein weiteres dialyseseitiges Schlauchsystem mit dem optischen Sensor 8 verbunden, der vorgegebene Parameter (Absorbanz, Absorbtion, etc.) der ausgangsseitigen, verbrauchten Dialysierflüssigkeit überwacht. Bei einer Änderung am Patienten, z.B. einer lokalen Rezirkulation, wie Shunt-Rezirkulation mit Herabsetzung der effektiven Clearance, muss sich diese Änderung erst über die Strecke bis zum optischen Sensor bemerkbar machen. Das bedeutet, dass der Transport durch den arteriellen Schlauchabschnitt, den Dialysator und durch die dialysierflüssigkeitsseitige Verschlauchung erfolgen muss, bevor die Auswirkung dieser Veränderung am Systemeingang (Patientenzugang) durch den optischen Sensor 8 detektiert werden kann. Diese Zeitverzögerung ist in Fig. 5B wiedergegeben.

In Fig. 5B ist der Signalverlauf über die Zeit aufgetragen. Aus Fig. 5B ergibt sich, dass die Auswirkung einer lokalen Rezirkulation am Patientenzugang, wie Shunt-Rezirkulation für den optischen Sensor 8 erst deutlich zeitverzögert messbar ist. Daher ist auch eine Wartezeit nach Abfrage 20 "Qb_ziel erreicht?" oder Abfrage 19 "PV oder PA erreicht?" vorgesehen vor der Abfrage 27 bzw. 28 "I_min bzw. A_max erreicht?", um zu vermeiden, dass der falsche Blutfluss eingestellt wird.

Das bedeutet, dass wie aus Fig. 5A und 5B ersichtlich systembedingt eine Änderung, welche am Patientenzugang/Shunt des Patienten auftritt, erst dann durch den optischen Sensor 8 detektiert werden kann, wenn sie das Schlausystem blutseitig, den Dialysator 4 und die dialysierflüssigkeitsseitige Strecke bis zum Sensor 8 durchlaufen hat. Daher wird ein Ereignis, das zum Zeitpunkt t auftritt, erst mit Verzögerung (Totzeit) zum Zeitpunkt t + Δt detektiert.

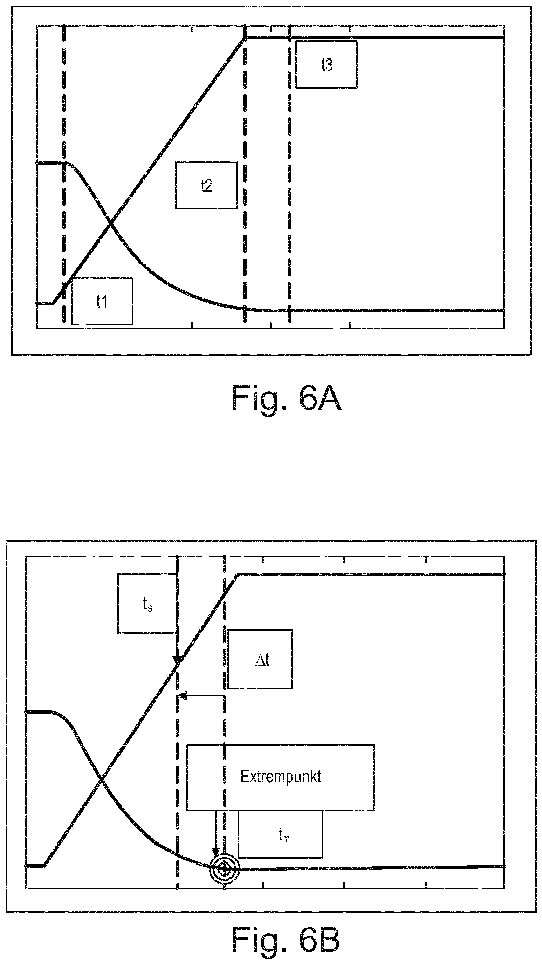

In Fig. 6A und Fig. 6B ist die Bestimmung des Blutflusses als Beispiel erläutert. In beiden Figuren 6A und 6B ist sowohl der Anstieg des Blutflusses (ansteigende Linie in Fig. 6A und 6B) als auch das Intensitätssignal des optischen Sensors 8 (abklingende Kurve in Fig. 6A und 6B) über die Zeit aufgetragen. In Fig. 6A wird der Blutfluss ab dem Zeitpunkt t1 erhöht. Der Zeitpunkt t2 kennzeichnet das Ende der Blutflusssteigerung, während bei t3 das Ende der Intensitätsänderung am optischen Sensor 8 erreicht wird. Da der optische Sensor 8 auf Dialysierflüssigkeitsseite angebracht ist, kommt es stets zu einer zeitlichen Verzögerung. Blutseitige Systemänderungen, die Auswirkung auf die Toxinmenge in der Dialysierflüssigkeit haben, sind nicht bei Auftreten sofort am optischen Sensor 8 messbar. Hier ist zusätzlich der Transport durch das blutseitige Schlauchsystem und den Dialysator 4 etc. zu berücksichtigen. Zusätzlich treten Ausgleichsvorgänge auf, die durch die Volumina (blutseitig, dialysierflüsigkeitsseitig) im Dialysator 4 bedingt sind.

In Fig. 6B ist die Verzögerungszeit im Signal aufgetragen. Durch die Blutflusserhöhung wurde hier zum Zeitpunkt ts eine Konzentrationsänderung am Patientenzugang induziert. Die Ursache für diese Konzentrationsänderung kann beispielsweise eine lokale Rezirkulation / Shunt-Rezirkulation sein. Hierdurch können weniger Substanzen den Weg auf die Dialysierflüssigkeitsseite finden. Tritt diese zum Zeitpunkt ts während der Blutflusserhöhung ein, beginnt zunächst die detektierte Intensität zu fallen um dann nach einer gewissen Zeit wieder zu steigen, obwohl der Blutfluss weiterhin erhöht wird (hier sei erwähnt, dass die Intensität in Folge der Blutflusserhöhung prinzipiell immer sinken sollte, nur der nachfolgende Anstieg würde das Insduzieren einer Rezirkulation bedeuten). In Fig. 6B zeigt sich diese Konzentrationsänderung durch Ausprägung eines Extrempunktes (zum Zeitpunkt tm) im Sensorsignal des optischen Sensors 8. Die bereits beschriebene Verzögerungszeit zwischen eingangsseitigem Auftreten und der ausgangseitigen Messung der Auswirkung ist in Fig. 6B als $\Delta t$ bezeichnet (und entspricht ggf. der Wartezeit tx, wobei idealer Weise gilt: $\Delta t$ kleiner/gleich tx).

Die Auftretenszeit tm dieses Extrempunktes steht in Zusammenhang mit der Zeit ts, zu der die Komplikation am Eingang auftritt. Existiert eine Änderung am Eingang, gilt:

$$tm > ts$$

$$ts = tm - \Delta t.$$

Ist $\Delta t$ bekannt, kann aus der Kenntnis von tm der optimale Behandlungsblutfluss ermittelt werden, da dieser der Blutfluss Qb (tm - $\Delta t$) ist.

Diese Totzeit $\Delta t$ ist abhängig von der gewählten Blutflussänderung Qb(t) bzw. Qb_ziel, dem Dialysierflüssigkeitsfluss Qd und den beteiligten Volumina z.B. im Dialysator 4 (Vbeff effektives blutseitiges Volumen, Vdeff effektives dialysierflüssigkeitsseitiges Volumen) und im Schlauchsystem.

Damit ergibt sich:

$$\Delta t = f(Qb(t), Qd, Vbeff, Vdeff, VSchlauch\_arteriell).$$

Sowohl Qb(t), Qd, Vbeff, Vdeff als auch VSchlauch_arteriell sind bekannte Größen, die z.B. aus Labormessungen oder Datenblättern entnommen wurden. Dazu wird der Dialysator 4 und das blutseitige Schlauchsystem vor der Behandlung identifiziert, um auf die relevanten Tabellen zugreifen zu können. Die Identifizierung des Dialysators 4 bzw. des Schlauchsystems erfolgt über die Kommunikationseinrichtung 16 durch den Benutzer, das Einlesen eines Barcodes oder Laden der Daten von einem Server, etc.

Will man $\Delta t$ nicht aus den Größen Qb, Qd, VSchlauch_arteriell, Vbeff und Vdeff errechnen, wird $\Delta t$ direkt in einer Tabelle hinterlegt, denn die Abhängigkeit von $\Delta t$ und Qb_ziel ist aus Labormessungen bekannt, wenn gleichzeitig der Dialysator 4 und das Schlauchsystem (z.B. durch Benutzereingabe) identifiziert worden sind. Qd muss ebenfalls bekannt sein. Der Dialysierflüssigkeitsfluss Qd ist jederzeit messtechnisch erfasst. Table 1 zeigt ein Beispiel für die Zuordnung der Werte von $\Delta t$ zu verschiedenen Qb_ziel bei einem Dialysierflüssigkeitsfluss von 500ml/min. In diesem Beispiel sind der Dialysator 4 und das Schlauchsystem sowie der Dialysierflüssigkeitsfluss Qd bereits bekannt. Daher wird auf eine Lookup-Tabelle zurückgegriffen, die die Werte von $\Delta t$ für die jeweilige Konfiguration von Dialysator 4 und Schlauchsystem sowie Qd enthält.

Tabelle 1

| Qb_ziel [ml/min] | $\Delta t$ [s] |
| --- | --- |
| 300 | 20 |
| 400 | 25 |

Mit der Erfindung wird erstmalig eine Vorrichtung geschaffen, die eine Einstellung des Blutflusses automatisch anhand der effektiven Clearance vornimmt und zusätzlich durch die Drucküberwachung sicherstellt, dass der Patient nicht gefährdet wird. Im Einzelnen bedeutet dies, dass die Situation am Patientenzugang z.B. beim Anlegen und dem Einstellen des Blutflusses unmittelbar (online) überwacht werden kann. Damit gleichzusetzen ist, dass jede blutflussinduzierte Minderung der effektiven Clearance (quasi-) instantan erfasst werden und somit der Blutfluss auf die maximale therapeutische Wirkung abgestimmt werden kann. Mit anderen Worten, es kann eine Optimierung der Therapie auf die maximale Clearance mit dem erfindungsgemäßen Verfahren erreicht werden. Ferner wird durch Analyse des Ausgleichsvorganges (Transientenmessung) auf Dialysierflüssigkeitsseite, der durch einen optischen Sensor gemessen wird, eine Regelung auf den optimalen Blutfluss durchgeführt. Selbst bei Komplikationen am Zugang kann die optimale Dialysebehandlung jedes Patienten sichergestellt werden. Durch Regeln/Steuern des Blutflusses auf die maximale Reinigungsleistung und damit die Möglichkeit, den Dialysierflüssigkeitsfluss zu reduzieren, bis der gewünschte Kt/V erreicht ist (angegeben durch Kt/V-Prädiktion), kann Dialysierflüssigkeit eingespart werden. Dies gilt insbesondere dann, wenn Kt/V über dem gewünschten Qualitätsniveau liegt. Ferner kann der vorstehend beschriebene Vorgang zu jeder Zeit auch innerhalb der Behandlung neu ausgelöst werden, wenn dies vom behandelnden Personal gewünscht ist (Die Blutflussänderung kann zu diesem Zweck in beide Richtungen, nämlich erhöhen/verringern erfolgen). Durch Speichern der gefunden Blutflüsse und die Trendauswertung kann die Shunt-Situation überwacht werden. Eine Rezirkulation wird automatisch durch Ermitteln des Extrempunktes erkannt. Die Messzeiten können kurz gehalten werden, so dass die Messdauer unter vier Minuten liegt. Aufgrund der bereits vorhandenen Sensorik lassen sich die zu erwartenden Kosten niedrig halten. Durch die Messung auf der Dialysierflüssigkeitsseite hat das Pflegepersonal keinen zusätzlichen Aufwand bei der Vorbereitung der Dialyse und dem Einlegen des Blutschlauchsystems in die Sensoren und wird zusätzlich durch eine automatische Anlegeprozedur entlastet.

In einer (nicht gezeigten) bevorzugten Ausführungsform wird ein Rotdetektor zum Erkennen von Blut beim Anlegen des Patienten eingesetzt. Es ist eine Blutpumpenrate von 50ml/min bis Patientenzugang erreichbar. Eine Blutflusserhöhung auf einen verschriebenen Blutflusswert kann automatisch oder manuell durch das Bedienerpersonal initiiert werden (in Fig. 6A und 6B auf Qb_ziel kleiner/gleich 600ml/min). Der optische Sensor überwacht die Intensität im Abfluss, und die Steuereinrichtung 15 wertet kontinuierlich das Signal aus und ermittelt z. B. die Steigung. Drucksensoren überwachen die zulässigen Druckgrenzen. Die Steuereinrichtung 15 wertet die Daten aus und bestimmt die Auftretenszeit eines Extremums oder ermittelt das Eintreten eines anderen Abbruchkriteriums (z.B. PV oder PA erreicht). Mit Hilfe einer Look-up Tabelle wie der Tabelle 1 wird aus tm - Δt der Wert von ts bestimmt. Für die Wahl der richtigen Lookup-Tabelle muss die Konfiguration von Dialysator und Schlauchsystem sowie Qd bekannt sein.

Die Erfindung besteht aus einer Vorrichtung und dem anhängigen Verfahren zur Bestimmung des optimalen Blutflusses vorzugsweise zu Therapiebeginn. Diese Messung kann ohne zusätzlichen apparativen Aufwand erfolgen, da die Dialysemaschine bereits über alle nötige Aktorik und Sensorik verfügt. Der Blutfluss, bei dem ein Maximum an urämischen Substanzen den Weg von der Blut- nach der Dialysierflüssigkeitsseite findet, wird bestimmt durch das Erreichen eines von drei Kriterien gemäß der vorstehenden Beschreibung, wenn der Blutfluss geändert wird. Ein Kriterium ist das Erreichen der Druckgrenzen PV bzw. PA, das andere Kriterium ist das Auffinden der Extremwerte I_min bzw. A_max und das letzte Kriterium ist schließlich das Erreichen des voreingestellten maximalen Blutflusses (Zielblutflusses). Da der Blutfluss mit dem erfindungsgemäßen Verfahren im wesentlichen an seinem Maximalwert gehalten werden kann, kann für die gleiche Reinigungsleistung der Dialysierflüssigkeitsfluss herabgesetzt werden, so dass sich damit ggf. ein Einsparpotential eröffnet (in der Regel dann, wenn sich während der Behandlung zeigt, dass die geforderte Dialysedosis übertroffen wird).

Die erfindungsgemäße Blutbehandlungsmaschine / Dialysemaschine umfasst folglich einen Dialysator 4 zum Blutreinigen. Zur Aufrechterhaltung eines externen Blutkreislaufs ist wenigstens eine Blutpumpe 7 vorgesehen, die einen Blutfluss zwischen einem Patienten und dem Dialysator 4 erzeugt. Auf der anderen Seite des Dialysators 4 ist wenigstens eine Dialysierflüssigkeitspumpe 2, 9 vorgesehen, mit der der Dialysator 4 mit einer Dialysierflüssigkeit versorgt wird. Zur Überwachung des Dialysevorgangs ist vorzugsweise wenigstens ein Venösblutdruckaufnehmer 5 unterhalb (stromab) des Dialysators 4 vorgesehen. Analog umfasst die Blutbehandlungsmaschine / Dialysemaschine vorzugsweise wenigstens einen Arteriellblutdruckaufnehmer 6 oberhalb (stromauf) des Dialysators 4 und vorzugsweise wenigstens einen Dialysierflüssigkeitssensor (optischen Sensor) 8 zum Erfassen wenigstens eines Dialysatparameters unterhalb (stromab) des Dialysators 4. Auf der Blutseite des Dialysators 4 wird vorzugsweise wenigstens ein Blutflusssensor 13 zum Erfassen eines Blutflusses eingesetzt. Ferner umfasst die Blutbehandlungsmaschine / Dialysemaschine vorzugsweise wenigstens einen Dialysierflüssigkeitsflusssensor 12 zum Erfassen eines Dialysierflüssigkeitsflusses.

Über eine Kommunikationseinrichtung 16 wird ein Blutflusszielwert, Qb_ziel, vorgegeben. Eine Steuereinrichtung 15 dient zum Einstellen eines optimalen Blutflusswertes in Abhängigkeit von dem voreingestellten Blutflusszielwertes, Qb_ziel, den erfassten oberen und unteren Blutdrücken PA, PV sowie der eventuellen Ermittelung eines Parameterextrems. Die Steuereinrichtung 15 weist erfindungsgemäß eine (nicht dargestellte) Blutpumpensteuereinheit zum Verändern des Blutflusses, Qb, mit einer vorgegebenen Blutflussänderungsrate oder ein Stromregelventil auf. Diese Rate beträgt insbesondere (Qb_ziel - Qb_start) /t. Eine (nicht dargestellte) Vergleichereinheit (innerhalb der Steuereinrichtung 15) dient zum Vergleichen eines venösen Drucks PV mit einem venösen Druckschwellenwert, eines arteriellen Drucks PA mit einem arteriellen Druckschwellenwert und des aktuellen Blutflusses Qb mit dem Blutflusszielwert Qb_ziel. Eine (nicht dargestellte) Ermittlungseinrichtung (Bestandteil der Steuereinrichtung 15) ist vorgesehen zum Ermitteln eines Parameterextrems in der verbrauchten Dialysierflüssigkeit aus den erfassten Parameterwerten vom Sensor 8. Ein (nicht dargestellter) Blutflussoptimalwertspeicher (ebenfalls Bestandteil der Steuereinrichtung 15) dient zum Abspeichern eines Blutflussoptimalwertes Qb_optimum beispielsweise in Abhängigkeit von jenem Blutfluss, der zum Zeitpunkt des Auftretens des Parameterextrems tatsächlich vorgeherrscht hat und bevorzugt in Abhängigkeit von in der Lookup Tabelle aufgeführten Daten zu Qb_ziel und Δt (tatsächliche Verzögerungszeit), wenn von der Ermittlungseinrichtung das Erreichen des Parameterextrems (Dialysierflüssigkeitsparameterschwellenwerts) ggf. innerhalb der Warte-/Verlängerungszeit tx erkannt worden ist oder wenn von der Vergleichereinheit das Erreichen des venösen Druckschwellenwerts oder des arteriellen Druckschwellenwerts erkannt worden ist und von der Ermittlungseinrichtung das Nichterreichen des Parameterextrems (Dialysierflüssigkeitsparameterschwellenwerts) ggf. auch innerhalb der Warte-/Verlängerungszeit tx erkannt worden ist. Wenn keine der obigen Bedingungen erfüllt ist, wird durch eine (nicht dargestellte) Rücksprungeinrichtung der Blutfluss, Qb, mit der vorgegebenen Blutflussänderungsrate wie vorher weiter verändert (erhöht), solange bis maximal der eingestellte Blutfluss Qb_ziel erreicht ist.

Vorzugsweise ist eine (nicht dargestellte) Verzögerungs-/Verlängerungseinheit (Bestandteil der Steuerungseinrichtung 15) zwischen der Vergleichereinheit und der Ermittlungseinrichtung/-einheit vorgesehen zum Hinauszögern/Verlängern des Ermittlungsvorgangs durch die Ermittlungseinrichtung um die (vorgegebene) Warte-/Verlängerungszeit tx, wenn von der Vergleichereinheit das Erreichen eines DruckSchwellenwerts vorerst erkannt worden ist, wie dies vorstehend bereits beschrieben wurde.

In anderen Worten ausgedrückt, setzt die Ermittlungseinheit nach Erreichen eines Druckschwellwerts den Ermittlungsvorgang um die Verlängerungszeit tx fort, um eine Entscheidung über das Vorliegen eines Parameterextrems an das Ende der Verlängerungszeit tx zu verzögern. Sollte dann festgestellt werden, dass kein Parameterextrem in der verbrauchten Dialysierflüssigkeit vorlag, bleibt er bei dem zunächst abgespeicherten Blutfluss bei dem der Druckschwellwert erreicht wurde. Sollte aber nachträglich das Vorliegen eines Parameterextrems verzögert erfasst werden, lässt sich anhand der vorab gespeicherten Lookup Tabelle der Blutfluss zum realen Zeitpunkt des Parameterextrems bestimmen, entsprechend dem dann der Blutflussoptimalwert definiert wird.

Vorzugsweise berechnet die Blutpumpensteuereinheit die Blutflussänderungsrate in Abhängigkeit von einem vorgegebenen Blutflussanfangswert Qb_start dem Blutflusszielwert Qb_ziel und einer vorgegebenen Blutflussänderungsdauer, t. Der Blutflusszielwert Qb_ziel kann als Defaultwert ausgelesen werden, von einer Kommunikationseinrichtung eingelesen werden, von einer Patientenkarte eingelesen werden oder von einem Server eingelesen werden.

Die Verzögerungs-/Verlängerungseinheit bestimmt vorzugsweise die Warte-/Verlängerungszeit tx in Abhängigkeit von der Blutflussänderungsrate, dem Blutflusszielwert Qb_ziel einem Dialysierflüssigkeitsfluss Qd und Parametern der Blutbehandlungsmaschine / Dialysemaschine. Insbesondere kann die Verzögerungseinheit die Warte-/Verlängerungszeit tx aus der Daten-/Wertetabelle (Lookup Tabelle) einlesen, wobei in der Daten-/Wertetabelle die Verzögerungszeit Δt und der Blutflusszielwert Qb_ziel als Wertepaare in Abhängigkeit von Parametern der Blutbehandlungsmaschine / Dialysemaschine abgespeichert sind, damit die Bedingung tx ≥ Δt erfüllt bleibt.

Bezugszeichenliste

1 Dialysierflüssigkeitszulauf
2 Dialysierflüssigkeitspumpe am Dialysierflüssigkeitseingang
3 Substitutionspumpe
4 Dialysator
5 Druckaufnehmer venös
6 Druckaufnehmer arteriell
7 Blutpumpe
8 Sensor, z.B. optischer Sensor
9 Dialysierflüssigkeitspumpe Dialysierflüssigkeitsausgang
10 Blutseite des Dialysators
11 Dialysierflüssigkeitsseite des Dialysators
12 Flusssensor Dialysierflüssigkeitsseite
13 Flusssensor Blutseite
14 Abfluss
15 Steuereinrichtung
16 Kommunikationseinrichtung
17 Vorgeben eines Blutflusszielwertes, Qb_ziel, über eine Kommunikationseinrichtung (16),
18 Verändern des Blutflusses, Qb, mit einer vorgegebenen Blutflussänderungsrate durch eine Blutpumpensteuereinheit,
19 Vergleichen eines venösen Drucks, PV, mit einem venösen Druckschwellenwert, eines arteriellen Drucks, PA, mit einem arteriellen Druckschwellenwert

20 Vergleichen des Blutflusses, Qb, mit dem Blutflusszielwert, Qb_ziel, vorzugsweise durch eine Vergleichereinheit,

21 Ermitteln eines Dialysierflüssigkeitsparameterschwellenwerts (Parameterextrems), d. h. Prüfen auf globalen Extremwert, was dem Kriterium "$I_{min}$ / $Abs_{max}$" entspricht, durch eine Ermittlungseinrichtung/Ermittlungseinheit,

22 Abspeichern eines Blutflussoptimalwertes, Qb_optimum,

23 Betreiben der Maschine mit dem Blutflussoptimalwertes, Qb_optimum, bis auf weiteres und Beenden des Verfahrens

24 Betreiben der Maschine mit dem Blutflusswert Qb_P_grenz - x% während einer vorgegebenen Wartezeit tx,

25 Ermitteln eines Dialysierflüssigkeitsparameterschwellenwerts (Parameterextrems), d. h. Prüfen auf globalen Extremwert, was dem Kriterium "$I_{min}$ / $Abs_{max}$" entspricht, durch eine Ermittlungseinrichtung/Ermittlungseinheit,

26 Berechnen des neuen Blutflusswertes Q_b

27 Betreiben der Maschine mit dem Blutflusswert Qb_ziel während einer vorgegebenen Wartezeit tx,

28 Ermitteln eines Dialysierflüssigkeitsparameterschwellenwerts (Parameterextrems), d. h. Prüfen auf globalen Extremwert, was dem Kriterium "$I_{min}$ / $Abs_{max}$" entspricht, durch eine Ermittlungseinrichtung/Ermittlungseinheit,

29 Berechnen des neuen Blutflusswertes Q_b

30 Berechnen des neuen Blutflusswertes Q_b

**Patentansprüche**

1. Extrakorporale Blutbehandlungsmaschine, die aufweist:

einen Dialysator (4),
wenigstens eine Blutpumpe (7) zum Erzeugen eines extrakorporalen Blutflusses zwischen einem Patienten und dem Dialysator (4),
wenigstens eine Dialysierflüssigkeitspumpe (2, 9) zum Versorgen des Dialysators (4) mit einer Dialysierflüssigkeit,
wenigstens einen venösen Blutdruckaufnehmer (5) stromab des Dialysators (4),
wenigstens einen arteriellen Blutdruckaufnehmer (6) stromauf des Dialysators (4),
wenigstens einen Dialysierflüssigkeitssensor (8) zum Erfassen wenigstens eines Dialysierflüssigkeitsparameters stromab des Dialysators (4) und
eine Steuereinrichtung (15) zum Einstellen eines Blutflusswertes des Blutflusses in Abhängigkeit von dem Blutflusszielwert (Qb_ziel), wobei
die Steuereinrichtung (15) aufweist:

eine Steuer-/Regeleinheit zum Verändern des aktuellen Blutflusses (Qb) mit einer vorgegebenen oder ausgewählten Blutflussänderungsrate,
eine Vergleichereinheit zum Vergleichen eines venösen Drucks (PV) mit einem venösen Druckschwellenwert, eines arteriellen Drucks (PA) mit einem arteriellen Druckschwellenwert und des aktuellen Blutflusses (Qb) mit dem Blutflusszielwert (Qb_ziel),
eine Ermittlungseinheit zum Ermitteln eines Dialysierflüssigkeits-Parameterextrems aus einer Anzahl von Messwerten durch den Dialysierflüssigkeitssensor (8),
einen Blutflussoptimalwertspeicher zum Abspeichern eines Blutflussoptimalwertes (Qb_optimum), wenn von der Ermittlungseinheit ein Dialysierflüssigkeits-Parameterextrem ermittelt worden ist oder wenn von der Vergleichereinheit das Erreichen des venösen Druckschwellenwerts, des arteriellen Druckschwellenwerts oder des Blutflusszielwerts erkannt worden ist und von der Ermittlungseinheit kein oder noch kein Dialysierflüssigkeits-Parameterextrem ermittelt worden ist, wobei die Blutbehandlungsmaschine dazu ausgelegt ist, folgende Schritte auszuführen:

a) Vorgeben (17) des Blutflusszielwertes (Qb_ziel);
b) Verändern (18) des Blutflusses (Qb) mit einer vorgegebenen oder ausgewählten Blutflussänderungsrate;
c) Vergleichen (19, 20) des venösen Drucks (PV) mit dem venösen Druckschwellenwert, des arteriellen Drucks (PA) mit dem arteriellen Druckschwellenwert und des aktuellen Blutflusses (Qb) mit dem Blutflusszielwert (Qb_ziel) durch die Vergleichereinheit,
d) Ermitteln des Dialysierflüssigkeits-Parameterextrems durch die Ermittlungseinheit,

wobei der Schritt d) (21) nach einer vorgegebenen Wartezeit (tx) (24, 27) nach Schritt c) (19, 20) ausgeführt wird, wenn in Schritt c) (19, 20) ein Schwellenwert erreicht wird, oder der Schritt d) (21) über eine bestimmte Verlängerungszeit (tx) (24, 27) nach Schritt c) (19, 20) hinaus getaktet oder kontinuierlich ausgeführt wird, wenn in Schritt c) (19, 20) ein Schwellenwert oder der Blutflusszielwert (Qb_ziel) erreicht wird.

2. Blutbehandlungsmaschine nach Anspruch 1, die als Dialysemaschine ausgebildet ist.

3. Blutbehandlungsmaschine nach Anspruch 1 oder 2, bei der eine Warte-/Verlängerungsdauer (tx) in Abhängigkeit von einer Verzögerungszeit ($\Delta$t) zwischen dem tatsächlichen Auftreten des Dialyseflüssigkeits-Parameterextrems und dessen Erfassen an einer Erfassungsstelle insbesondere in Abhängigkeit von der Blutflussänderungsrate, dem Blutflusszielwert (Qb_ziel), einem Dialysierflüssigkeitsfluss (Qd) und Parametern der Blutbehandlungsmaschine vorgegeben oder einstellbar ist, und die Verzögerungszeit ($\Delta$t) und der Blutflusszielwert (Qb_ziel) als Wertepaare in einer Lookup-Wertetabelle abgespeichert sind.

4. Blutbehandlungsmaschine nach einem der vorhergehenden Ansprüche, bei der die Steuereinrichtung (15) ferner aufweist:

   eine Verzögerungs-/Verlängerungseinheit zum Verzögern/Verlängern des Ermittlungsvorgangs durch die Ermittlungseinheit mindestens um eine vorgegebene Warte-/Verlängerungszeit (tx), wenn von der Vergleichereinheit das Erreichen eines Schwellenwerts erkannt und vom Blutflussoptimalwertspeicher ein zughöriger Blutfluss als Blutflussoptimalwert (Qb_optimum) temporär abgespeichert worden ist.

5. Blutbehandlungsmaschine nach einem der vorhergehenden Ansprüche, bei der der Blutflussoptimalwert (Qb_optimum) in Abhängigkeit von einem neuen Blutflusswert (Q_b) oder in Abhängigkeit des Blutflusszielwerts (Qb_ziel) entsprechend einer vorab gespeicherten Datentabelle ermittelt sowie in dem Blutflussoptimalwertspeicher abgespeichert wird, wenn die Ermittlungseinheit während oder nach der Warte-/Verlängerungszeit (tx) ein Dialysierflüssigkeits-Parameterextrem ermittelt, wobei der neue Blutflusswert (Q_b) dem Blutfluss entspricht, der bei Auftreten des Parameterextrems tatsächlich vorgeherrscht hat.

6. Blutbehandlungsmaschine nach einem der vorhergehenden Ansprüche, bei dem die Steuereinrichtung (15) die Blutflussänderungsrate in Abhängigkeit von einem vorgegebenen Blutflussanfangswert (Qb_start) dem Blutflusszielwert (Qb_ziel) und einer vorgegebenen Blutflussänderungsdauer (t) berechnet.

7. Blutbehandlungsmaschine nach einem der vorhergehenden Ansprüche, bei dem die Steuereinrichtung (15) den vorgegebenen Blutflussanfangswert (Qb_start) auf 50ml/min festsetzt, wobei der Blutflusszielwert (Qb_ziel) als Defaultwert in der Steuereinrichtung (15) abgelegt ist, oder von einer Kommunikationseinrichtung (16), von einer Patientenkarte einliest oder von einem Server eingelesen wird.

8. Blutbehandlungsmaschine nach Anspruch 4, bei der die Verzögerungs-/Verlängerungseinheit die Warte-/Verlängerungszeit (tx) in Abhängigkeit von der Blutflussänderungsrate, dem Blutflusszielwert (Qb_ziel) einem Dialysierflüssigkeitsfluss (Qd) und Parametern der Blutbehandlungsmaschine bestimmt oder die Warte-/Verlängerungszeit (tx) in Abhängigkeit von der Verzögerungszeit ($\Delta$t) zwischen dem tatsächlichen Auftreten des Dialyseflüssigkeits-Parameterextrems und dessen Erfassen an einer Erfassungsstelle aus einer vorabgespeicherten Daten- oder Wertetabelle einliest, wobei in der Wertetabelle die Verzögerungszeit ($\Delta$t) und der Blutflusszielwert (Qb_ziel) als Wertepaare in Abhängigkeit von Parametern der Blutbehandlungsmaschine abgespeichert sind.

9. Blutbehandlungsmaschine nach einem der vorhergehenden Ansprüche, bei der das Vorgeben des Blutflusszielwertes (Qb_ziel) über eine Kommunikationseinrichtung (16) erfolgt.

10. Blutbehandlungsmaschine nach einem der vorhergehenden Ansprüche, bei der das Verändern der Blutflussänderungsrate durch eine Blutpumpensteuereinheit erfolgt.

**Claims**

1. An extracorporeal blood treatment machine which comprises:

   a dialyzer (4),
   at least one blood pump (7) for creating an extracorporeal blood flow between a patient and the dialyzer (4),
   at least one dialysis fluid pump (2, 9) for supplying the dialyzer (4) with a dialysis fluid,

at least one venous blood pressure sensor (5) downstream of the dialyzer (4),

at least one arterial blood pressure sensor (6) upstream of the dialyzer (4),

at least one dialysis fluid sensor (8) for detecting at least one dialysis fluid parameter downstream of the dialyzer (4), and

a control device (15) for adjusting a blood flow value of the blood flow in dependence on the blood flow target value (Qb_target), wherein

the control device (15) comprises:

a control/regulating unit for altering the current blood flow (Qb) at a predetermined or selected blood flow alteration rate,

a comparator unit for comparing a venous pressure (PV) with a venous pressure threshold, an arterial pressure (PA) with an arterial pressure threshold, and the current blood flow (Qb) with the blood flow target value (Qb_target),

a determination unit for determining a dialysis fluid parameter extreme from a number of measurement values through the dialysis fluid sensor (8),

an optimum blood flow value memory for storing an optimum blood flow value (Qb_optimum), if a dialysis fluid parameter extreme has been determined by the determination unit, or if the comparator unit recognized that the venous pressure threshold, the arterial pressure threshold or the blood flow target value has been reached and the determination unit has not, or not yet determined a dialysis fluid parameter extreme, wherein the blood treatment machine is adapted to execute the following steps:

a) predetermining (17) the blood flow target value (Qb_target),

b) altering (18) the blood flow (Qb) at a predetermined or selected blood flow alteration rate,

c) comparing (19, 20) the venous pressure (PV) with the venous pressure threshold, the arterial pressure (PA) with the arterial pressure threshold, and the current blood flow (Qb) with the blood flow target value (Qb_target) by means of the comparator unit,

d) determining the dialysis fluid parameter extreme through the determination unit,

wherein, after a predetermined waiting time (tx) (24, 27), step d) (21) is executed after step c) (19, 20), if a threshold is reached in step c) (19, 20), or wherein step d) (21) is executed, in a clocked mode or continuously, over a predetermined extension time (tx) (24, 27) after step c) (19, 20), if a threshold or the blood flow target value (Qb_target) is reached in step c) (19, 20).

2. Blood treatment machine according to claim 1, wherein it is configured as a dialysis machine.

3. The blood treatment machine according to claim 1 or 2, wherein a waiting/extension period (tx) is predetermined or adjustable in dependence on a delay time ($\Delta t$) between the actual occurrence of the dialysis fluid parameter extreme and its detection at a detection site, in particular in dependence on the blood flow alteration rate, the blood flow target value (Qb_target), a dialysis fluid flow (Qd) and parameters of the blood treatment machine, and the delay time ($\Delta t$) and the blood flow target value (Qb_target) are stored as pairs of values in a lookup table.

4. The blood treatment machine according to one of the preceding claims, wherein the control device (15) additionally comprises:

a delay/extension unit for delaying/extending the determination process of the determination unit by at least a predetermined waiting/extension time (tx), if the comparator unit recognized that a threshold has been reached and if an associated blood flow has been temporarily stored as an optimum blood flow value (Qb_optimum) by the optimum blood flow value memory.

5. The blood treatment machine according to one of the preceding claims, wherein the optimum blood flow value (Qb_optimum) is determined in dependence on a new blood flow value (Q_b) or in dependence on the blood flow target value (Qb_target) according to a previously stored data table and is stored in the optimum blood flow value memory, if the determination unit determines a dialysis fluid parameter extreme during or after the waiting/delay time (tx), the new blood flow value (Q_b) corresponding to the blood flow that actually prevailed at the time of occurrence of the parameter extreme.

6. The blood treatment machine according to one of the preceding claims, wherein the control device (15) calculates the blood flow alteration rate in dependence on a predetermined blood flow start value (Qb_start), the blood flow target value (Qb_target) and a predetermined blood flow alteration period (t).

7. The blood treatment machine according to one of the preceding claims, wherein the control device (15) sets the predetermined blood flow start value (Qb_start) to 50ml/min, the blood flow target value (Qb_target) being stored as a default value in the control device (15), or read-in by a communication unit (16), or read-in from a patient data card or from a server.

8. The blood treatment machine according to claim 4, wherein the delay/extension unit determines the waiting/extension time (tx) in dependence on the blood flow alteration rate, the blood flow target value (Qb_target), a dialysis fluid flow (Qd) and parameters of the blood treatment machine, or reads-in the waiting/extension time (tx) from a previously stored data or value table in dependence on the delay time ($\Delta t$) between the actual occurrence of the dialysis fluid parameter extreme and its detection at a detection site, the delay time ($\Delta t$) and the blood flow target value (Qb_target) being stored in said value table as pairs of values in dependence on parameters of the blood treatment machine.

9. The blood treatment machine according to one of the preceding claims, wherein the blood flow target value (Qb_target) is predetermined via a communication unit (16).

10. The blood treatment machine according to one of the preceding claims, wherein altering of the blood flow alteration rate occurs through a blood pump control unit.

**Revendications**

1. Machine de traitement extracorporel du sang, qui présente :

un dialyseur (4),
au moins une pompe à sang (7) pour produire un débit sanguin extracorporel entre un patient et le dialyseur (4),
au moins une pompe à liquide de dialyse (2, 9) pour alimenter le dialyseur (4) en un liquide de dialyse,
au moins un transducteur de pression veineuse (5) en aval du dialyseur (4),
au moins un transducteur de pression artérielle (6) en amont du dialyseur (4),
au moins un capteur de liquide de dialyse (8) pour détecter au moins un paramètre du liquide de dialyse en aval du dialyseur (4) et
un dispositif de commande (15) pour ajuster une valeur de débit sanguin d'un débit sanguin en fonction de la valeur cible de débit sanguin (Qb_ziel), dans laquelle le dispositif de commande (15) présente :

une unité de commande/régulation pour modifier le débit sanguin (Qb) instantané avec une vitesse prédéfinie ou sélectionnée de modification du débit sanguin,
une unité de comparaison pour comparer une pression veineuse (PV) à une valeur seuil de la pression veineuse, une pression artérielle (PA) à une valeur seuil de la pression artérielle et le débit sanguin (Qb) instantané à la valeur cible de débit sanguin (Qb_ziel),
une unité de détermination pour déterminer un extrême du paramètre du liquide de dialyse à partir d'un nombre de valeurs de mesure par le capteur de liquide de dialyse (8),
une mémoire des valeurs optimales de débit sanguin pour stocker la valeur optimale de débit sanguin (Qb_optimum) quand un extrême du paramètre du liquide de dialyse a été déterminé par l'unité de détermination ou quand le fait d'atteindre la valeur seuil de la pression veineuse, la valeur seuil de la pression artérielle ou la valeur cible de débit sanguin a été reconnu par l'unité de comparaison et qu'aucun ou encore aucun extrême du paramètre du liquide de dialyse n'a été déterminé par l'unité de détermination, dans laquelle la machine de traitement du sang est conçue pour exécuter les étapes suivantes :

a) la prédéfinition (17) de la valeur cible de débit sanguin (Qb_ziel) ;
b) la modification (18) du débit sanguin (Qb) à une vitesse prédéfinie ou sélectionnée de modification du débit sanguin ;
c) la comparaison (19, 20) de la pression veineuse (PV) à la valeur seuil de la pression veineuse, de la pression artérielle (PA) à la valeur seuil de la pression artérielle et du débit sanguin (Qb) instantané à la valeur cible de débit sanguin (Qb_ziel) par l'unité de comparaison,
d) la détermination, par l'unité de détermination, de l'extrême du paramètre du liquide de dialyse,

dans laquelle l'étape d) (21) est exécutée après un temps d'attente (tx) prédéfini (24, 27) après l'étape c) (19, 20), quand dans l'étape c) (19, 20) une valeur seuil est atteinte, ou l'étape d) (21) est exécutée d'une manière cadencée ou continue sur un certain temps de prolongement (tx) (24, 27) après l'étape c) (19, 20), quand dans l'étape c) (19, 20) une valeur seuil ou la valeur cible de débit sanguin (Qb_ziel) est atteinte.

2. Machine de traitement du sang selon la revendication 1, qui est réalisée comme une machine de dialyse.

3. Machine de traitement du sang selon la revendication 1 ou 2, dans laquelle une durée d'attente/de prolongement (tx) est prédéfinie ou ajustable en fonction d'un temps de retard ($\Delta t$) entre l'apparition effective de l'extrême du paramètre du liquide de dialyse et sa détection en un point de détection, en particulier en fonction de la vitesse de modification du débit sanguin, de la valeur cible de débit sanguin (Qb_ziel), d'un débit de liquide de dialyse (Qd) et de paramètres de la machine de traitement du sang, et le temps de retard ($\Delta t$) et la valeur cible de débit sanguin (Qb_ziel) sont stockés en tant que paires de valeurs dans une table de correspondance.

4. Machine de traitement du sang selon l'une des revendications précédentes, dans laquelle le dispositif de commande (15) présente en outre :

une unité de retard/de prolongement pour retarder/prolonger d'au moins un temps d'attente/de prolongement (tx) prédéfini le processus de détermination par l'unité de détermination, quand le fait d'atteindre une valeur seuil a été reconnu par l'unité de comparaison, et qu'un débit sanguin correspondant a été provisoirement stocké, en tant que valeur optimale de débit sanguin (Qb_optimum) par la mémoire des valeurs optimales de débit sanguin.

5. Machine de traitement du sang selon l'une des revendications précédentes, dans laquelle la valeur optimale de débit sanguin (Qb_optimum) est déterminée en fonction d'une nouvelle valeur de débit sanguin (Q_b) ou en fonction de la valeur cible de débit sanguin (Qb_ziel) d'une manière correspondant à un tableau de données stockées au préalable, ainsi que stockée dans la mémoire des valeurs optimales de débit sanguin, quand l'unité de détermination détermine, pendant ou après le temps d'attente/de prolongement (tx), un extrême du paramètre du liquide de dialyse, dans laquelle la nouvelle valeur de débit sanguin (Q_b) correspond au débit sanguin qui a effectivement prévalu lors de l'apparition de l'extrême du paramètre.

6. Machine de traitement du sang selon l'une des revendications précédentes, dans laquelle le dispositif de commande (15) calcule la vitesse de modification du débit sanguin en fonction d'une valeur initiale de débit sanguin (Qb_start) prédéfinie, de la valeur cible de débit sanguin (Qb_ziel) et d'une durée de modification de débit sanguin (t) prédéfinie.

7. Machine de traitement du sang selon l'une des revendications précédentes, dans laquelle le dispositif de commande (15) fixe à 50 ml/min la valeur initiale de débit sanguin (Qb_start) prédéfinie, dans laquelle la valeur cible de débit sanguin (Qb_ziel) est déposée en tant que valeur par défaut dans le dispositif de commande (15), ou est introduite à partir d'un dispositif de communication (16), d'une carte patient ou d'un serveur.

8. Machine de traitement du sang selon la revendication 4, dans laquelle l'unité de retard/de prolongement détermine le temps d'attente/de prolongement (tx) en fonction de la vitesse de modification du débit sanguin, de la valeur cible de débit sanguin (Qb_ziel), d'un débit de liquide de dialyse (Qd) et de paramètres de la machine de traitement du sang, ou introduit le temps d'attente/de prolongement (tx) à partir d'un tableau de données et de valeurs stocké au préalable, en fonction du temps de retard ($\Delta t$) entre l'apparition effective de l'extrême du paramètre du liquide de dialyse et sa détection en un point de détection, dans laquelle le temps de retard ($\Delta t$) et la valeur cible de débit sanguin (Qb_ziel) sont, dans le tableau des valeurs, stockés en tant que paires de valeurs en fonction de paramètres de la machine de traitement du sang.

9. Machine de traitement du sang selon l'une des revendications précédentes, dans laquelle la prédéfinition de la valeur cible de débit sanguin (Qb_ziel) est effectuée par l'intermédiaire d'un dispositif de communication (16).

10. Machine de traitement du sang selon l'une des revendications précédentes, dans laquelle la modification de la vitesse de modification du débit sanguin est effectuée par une unité de commande de la pompe à sang.

Fig. 1

Clearance und Rezirkulation

Ce für R ≠ 0

Ce = Cd für R = 0

$Q_b$ /mL/min

Fig. 2

Maximum Effizienz im eingeschwungen System
—◆— Shuntkomplikation vorhanden

Qb / mL/min

Fig. 3

Hauptprogramm 1

( Start )
| Bediener, Default

/ Qb_ziel = Blutfluss
  Ziel / ——17

Maschine erhöht Qb mit
(Qb_Ziel-Qb_Start-)/t ——18

oberer PV
oder unterer PA
Grenzwert erreicht? — 19 → ja → Maschine ==>
Qb_P_grenz - x% =
konstant für eine Zeit tx — 24 → I_min bzw.
A_max erreicht? — 25 → nein ... ja → Berechne Q_b — 26

nein

Qb_ziel
erreicht? — 20 → ja → Maschine ==>Qb_ziel =
konstant für eine Zeit tx — 27 → I_min bzw.
A_max erreicht? — 28 → ja → Berechne Q_b — 29

nein                                          nein

I_min bzw.
A_max erreicht? — 21 → ja → Berechne Q_b — 30

nein

/ optimaler Blutfluss =
  Qb_optimum / — 22

Maschine stellt
Qb_optimum ein — 23

( Ende )

EP 2 985 045 B1

Fig. 4

| Eingang | $\longrightarrow$ | Ausgang |
|---------|-------------------|---------|

Schlauch                    Schlauch

| Patient | → | Dialysator | → | optischer Sensor |

# Fig. 5A

Komplikationen im Patienten

Δt

Auftreten im Sensorsignal

# Fig. 5B

Fig. 6A

Fig. 6B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5863421 A
- WO 2007140993 A
- US 3882861 A
- EP 0711182 B1
- EP 1083948 B1
- WO 2013167264 A